(19) Europäisches Patentamt
European Patent Office
Office européen des brevets

(11) Publication number: 0 530 866 A1

# (12) EUROPEAN PATENT APPLICATION

(21) Application number: 92202054.0

(51) Int. Cl.5: C07C 305/06, A61K 7/48

(22) Date of filing: 07.07.92

A request for correction of formatting errors in the description and claims has been filed pursuant to Rule 88 EPC. A decision on the request will be taken during the proceedings before the Examining Division (Guidelines for Examination in the EPO, A-V, 2.2).

(30) Priority: 30.07.91 US 738082
30.07.91 US 737962

(43) Date of publication of application:
10.03.93 Bulletin 93/10

(84) Designated Contracting States:
AT BE CH DE DK ES FR GB GR IT LI NL PT SE

(71) Applicant: UNILEVER N.V.
Weena 455
NL-3013 AL Rotterdam(NL)
(84) BE CH DE DK ES FR GR IT LI NL PT SE AT

(71) Applicant: UNILEVER PLC
Unilever House Blackfriars P.O. Box 68
London EC4P 4BO(GB)
(84) GB

(72) Inventor: Cho, Suk Hyung
670 Tilden Avenue
Teaneck, New Jersey 07026(US)

(74) Representative: van Gent, Jan Paulus et al
Unilever N.V., Patent Division Postbus 137
NL-3130 AC Vlaardingen (NL)

## (54) Alkyl sulphooxyalkanoate compounds and compositions.

(57) The invention relates to novel sulfooxy alkanoates surfactants

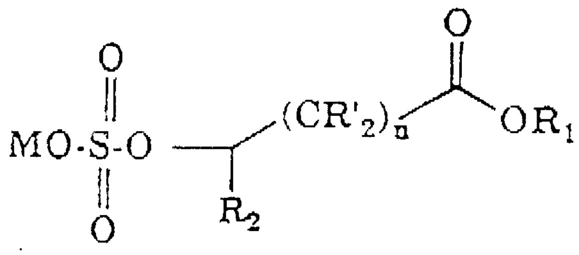

in which a portion of the surfactant molecule has a so-called "benefit reagent" function, and compositions containing these surfactants. It is believed that this benefit reagent portion comes to play when the surfactant metabolized or hydrolyzed.

The surfactants used in these compositions are calcium ion insensitive, show mildness benefits and foam well. Thus the surfactants have been demonstrated to be useful surfactant in addition to delivering a beneficial reagent.

EP 0 530 866 A1

## Background of the Invention

### 1. Field of the Invention

This invention relates to novel personal product compositi as well as to alkyl sulfooxyalkanoate surfactants used in compositions and methods for the preparation of these surfactants. A portion of the surfactant molecule functio as a so-called "benefit reagent" which will form when the molecule is metabolized by enzymes present in the body or elsewhere or which will form when the molecule spontaneous hydrolyzes on the skin surface. Personal products compositions comprising these sulfooxyalkanoate compounds include hair/body shampoos, cleansing creams, conditioners cosmetic applications, dental applications, underarm deodorant/antiperspirant applications and sunblock applications. Other applications include soaps, powders, lotions and therapeutic creams. This list is not intended be exhaustive and other compositions in which surfactants be used are also contemplated.

### 2. Prior Art

In the past decades, "mildness" has become an increasingly important criterion in selecting surfactants for personal products. The term "mildness" means that surfactants do n produce any skin irritation. Many consumers recognize the skin damaging effects surfactants may have. Therefore, a surfactant used for personal products should not only poss good surface active properties, but should also be safe on human skin.

Although many factors, e.g., removal of skin lipids, loss naturally occurring hygroscopic materials in the stratum corneum, adsorption, protein denaturation, epidermal liposomal injury, are known to have an influence on skin irritation, it is generally believed that surfactants caus skin irritation by penetrating the stratum corneum and reacting with the inner cells of the epidermis. According one approach to achieving "mildness" is to prevent surfactants from penetrating the stratum corneum and react with those cells.

A second approach for obtaining "mildness" is to design surfactants which can penetrate the stratum corneum but which, once they have penetrated, degrade to harmless components, possibly with the aid of enzymes. This second approach (the approach followed by the subject invention) attempts to take advantage of the enzymatic activity which believed to be present in the sublayer of the stratum corneum. See Foster et al, Arch Derm.Res., 25:23-28 (1975 and Kermici et al, J. Soc. Cosmet Chem., 28:151-164 (1977)

It is also known in the art that hydroxy acids have beneficial effects on the skin. U.S. Patent No. 4,197,316 Yu et al. for example, discloses a non-irritating therapeu composition for alleviating dry skin symptoms wherein the composition contains hydroxy acids (e.g., $\alpha$-hydroxy butyri acid, lactic acid and citric acid). U.S. Patent No. 4,294,852 to Wildnaer et al. teaches skin treating compositions comprising hydroxy acids. U.S. Patent No. 4,507,319 to Barret et al. also describes skin treatment compositions possessing $\alpha$-hydroxy octanoate acid.

None of these patents teach a surfactant material which is able to deliver the hydroxy acid component to the skin.

Accordingly, it would be particularly beneficial to design compositions comprising a surfactant molecule wherein the surfactant not only breaks down inside the skin or which spontaneously hydrolyzes upon contact with the skin surfac but to design a molecule which, once it has been broken do in the skin or once it has hydrolyzed, will have a benefic effect on (e.g., alleviating dryness, imparting antimicrob activity, etc.) or will deliver a benefit to the skin.

In U.S. Patent application No. 628,243, filed December 17, 1990 and hereby incorporated by reference, the inventor of the subject application provides novel compositions comprising phosphate esters which are designed to break do or hydrolyze and to impart the beneficial reagent componen described above. There is no description of compositions comprising the sulfooxy analogs of the present invention i that disclosure.

Peliska et al. in Biochemistry, 28: 1604-11 (1989), disclo simple sulfoenol pyruvate compounds prepared in order to study pyruvate kinase. The compounds are short-chained pyruvates which have no utility as surfactant materials. Further, there is no teaching at all that these compounds be used as intermediates to prepare a beneficial reagent o of their use in compositions.

Certain commercially available sulfosuccinic alkyl esters similar to the compounds of the invention. For example, Caryl, C. R., Industrial and Eng. Chem., 33 (6):731 (1941) teaches compounds in which an alkyl ester of succinic acid attached to a sulfonate group. These compounds are both structurally and chemically distinct from the sulfooxyalkanoate esters of the invention in that they comprise a sulfonate group rather than a sulfate group. T sulfate group is much more difficult to hydrolyze and will not accordingly readily

provide a beneficial reagent such is hypothesized is occurring with the compounds of the invention.

Stirton et al., Journal Amer. Oil Chem. Soc., 39: 490 (196 teaches alkyl sulfoalkanoate compounds. Smith et al., Journal Amer Oil Chem. Soc., 44:405 (1967) teaches α-sulfo fatty acid ester compounds. Once again, both of these references teach sulfonate compounds rather than sulfate compounds. Sulfonate compounds are far less soluble and structurally unrelated to the sulfate compounds of the invention.

None of the compounds taught in the art are sulfate surfactant compounds which are readily able to hydrolyze a from a beneficial reagent in the skin.

Thus, it would be useful to provide novel molecules (other than the phosphate esters mentioned above) and composition therefor, which contain a salt (i.e., at least a partial s on one portion and an ester group in the other portion) su that (1) a hydroxy carboxylic acid ester; (2) a hydroxy carboxylic acid; (3) a sulfooxy carboxylate; and/or (4) an alcohol (e.g., fatty acid alcohol or glycerol) may form wh the molecule in the composition is metabolized by enzymes the skin or is hydrolyzed upon contact with the skin..

It is a further objective of the invention to provide compositions containing beneficial reagent containing surfactants and compositions therefor which, as surfactant are also relatively calcium insensitive, may foam well and are mild to the skin.

## SUMMARY OF THE INVENTION

The present invention relates to novel surfactant molecule which are alkyl sulfooxy alkanoates, and to personal produ compositions which may contain these surfactants. These surfactants and compositions are useful in that they are mild, they may foam well, they are calcium insensitive, an they provide a beneficial reagent component to the skin. personal product compositions comprising these surfactants may be soap for compositions, body or facial cleaning compositions or toothpaste compositions, among others.

The sulfooxy alkanoate is a salt (or partial salt) in whic hydrogen, an alkali metal an alkaline earth metal, ammoniu alkyl ammonium, alkanolamine, cationic amino acids (e.g., arginine), or other salt forming cation is attached to one the single bonded sulphur oxygens and in which an alkyl gr is attached to the other single bonded sulphur oxygens, wherein the alkyl group contains an ester group. While no wishing to be bound by theory, it is hypothesized that the ester-containing group attached to the sulfoxy oxygen is capable of forming (1) a hydroxy carboxylic acid ester (if cleaved only at the left of the oxygen bonded to the sulfe atom); (2) a hydroxy carboxylic acid (if cleaved at the le of the oxygen bonded to the sulphur and between the carboxylate oxygen and the alkyl group attached to the carboxylate oxygen; (3) a sulfooxy carboxylate (if cleaved only between the carboxylate oxygen and the alkyl group attached to the carboxylate oxygen; and/or (4) a free alco such as fatty alcohol or glycerol (as a by-product of the cleavage of the carboxylate oxygen and the alkyl group) wh the sulfooxy ester surfactant molecule is metabolized or hydrolyzed.

Preferably, the final product after metabolism or hydrolysis is the hydroxy acid ester.

More particularly, the sulfate ester molecule is defined by formula I below:

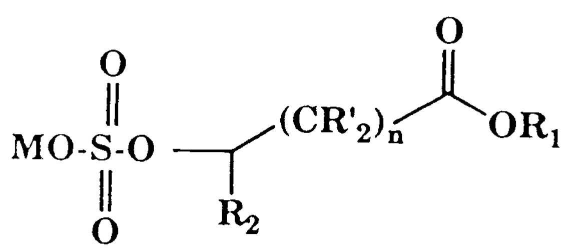

wherein:

M is hydrogen, an alkali metal such as lithium, sodium or potassium, an alkaline earth metal such as calcium, magnesium or strontium, ammonium, alkyl ammonium wherein the alkyl group is preferably a straight chain group having 1 to 22 carbons, alkanolamine, a cationic amino acid such as arginine or other salt forming cation such as, for example, a substituted pyridinium;

R' is hydrogen, a straight-chain alkyl group having 1 to 30 carbons, a branch-chain alkyl group having 4 to 30 carbons, or an organic moiety containing a functional group such as an ester, an acid, a hydroxyl group, a halide, an amide, or ammonium or a combination of any of the above (for example, one R' group may be hydrogen and another R' group may be methyl);

$R_2$ is hydrogen, a straight chain alkyl group having 1 to 30 carbons wherein any of the carbons may be substituted with a functional group such as an ester, hydroxy group (e.g., glycerol), halides, hydroxy fatty

amide (e.g., ceramides), or with a substituted or unsubstituted hydroxy fatty acid (e.g., -hydroxy fatty acid such as -hydroxy-6-cis- dodecenoic acid); a saccharide group such as monosaccharides (e.g., glucose), oligosaccharides (e.g., maltose) or mono- or oligosaccharides substituted with an alkyl chain having 1- carbons (e.g., alkylglucosides), the alkylglucosides being preferred to the other saccharides because the long hydrophobic tail allows them to act better as a surfactant group; a branched chain alkyl having 4 to 30 carbons; a straight or branched chain alkyl aryl group (preferably al phenyl) wherein the alkyl group may comprise 1-18 carbon atoms and wherein said alkyl aryl group may be condensed w a 2-5 carbon alkylene oxide; an aliphatic group having 6 t 30 carbons condensed with a 2-5 carbon alkylene oxide (encompassing the group of condensed alkyl aryl groups); a straight or branched-chain fluoroalkyl group having 5 to 2 carbons; a carbocyclic containing group such as cholestero retinol or hydroxy substituted retinoic acid; wherein any the alkyl groups described above may be linked by an ester group, amide, quaternary ammonium or heteroatom such as sulphur, oxygen or nitrogen;

$R_1$ may be the same or different than $R_2$; and

n = 0 to 50, preferably 0 to 10, most preferably 0 to 5.

The invention is further concerned with methods for produc the above-identified molecule.

## DETAILED DESCRIPTION OF THE INVENTION

The present invention provides novel sulfooxy alkanoate surfactants which are designed to deliver certain benefit enhancing agents (e.g., moisturizers) to the skin, and personal products compositions therefor. Since it is known that hydroxy acids, e.g., hydroxy caprylic acid (HCA), lac acid and analogs thereof provide a softening or plasticizi effect on skin, (see also U.S. Patent No. 4,197,316; and H et al. J. Soc. Cosmet Chem., 37: 397-407 (1986)) the surfactant molecules of the invention have been designed t incorporate a component which may, upon being metabolized hydrolyzed, form a hydroxy carboxylic acid ester, a hydrox carboxylic acid, a sulfooxy carboxylate and/or a free alco such as a fatty acid alcohol or glycerol. Preferably, the surfactant will metabolize or hydrolyze to form the hydrox carboxylic acid or alcohol.

More particularly, the surfactant molecule is defined by t formula:

wherein:

M is hydrogen, an alkali metal such as lithium, sodium or potassium, an alkaline earth metal such as calcium, magnes or strontium, ammonium, alkyl ammonium wherein the alkyl group is preferably a straight chain alkyl having 1 to 22 carbons, alkanolamine, a cationic amino acid such as argin or other salt forming cation such as, for example, a substituted pyridinium;

R' is hydrogen a straight-chain alkyl group having 1 to 30 carbons or a branched-chain alkyl group having 4 to 30 carbons, an organic moiety containing a functional group s as an ester, an acid, a hydroxyl group, a halide an amide ammonium, or a combination of any of the above (for exampl one R' group may be defined as a hydrogen while the other group is defined as methyl or ethyl etc.);

$R_2$ is hydrogen, a straight chain alkyl group having 1 to 3 carbons wherein any of the carbons may be substituted with functional group such as an ester, hydroxy group (e.g., glycerol), halides, hydroxy fatty amide (e.g., ceramides), with a substituted or unsubstituted hydroxy fatty acid (e. -hydroxy fatty acid such as -hydroxy-6-cis- dodecenoic acid); a saccharide group such as monosaccharides (e.g., glucose), oligosaccharides (e.g., maltose) or mono- or oligosaccharides substituted with an alkyl chain having 1-carbons (e.g., alkylglucosides), the alkylglucosides being preferred to the other saccharides because the long hydrophobic tail allows them to act better as a surfactant group; a branched chain alkyl having 4 to 30 carbons; a straight or branched chain alkyl aryl group (preferably al phenyl) wherein the alkyl group may comprise 1-18 carbon atoms and wherein said alkyl aryl group may be condensed w a 2-5 carbon alkylene oxide; an aliphatic group having 6 t 30 carbons condensed with a 2-5 carbon alkylene oxide (encompassing the group of condensed alkyl aryl groups); a straight or branched-chain fluoroalkyl group having 5 to 2 carbons; a carbocyclic containing group such as cholestero retinol or hydroxy substituted retinoic acid; wherein any the alkyl groups described above may be linked by an ester group, amide, quaternary ammonium or heteroatom such as sulfur, oxygen or nitrogen;

$R_1$ may be the same or different than $R_2$; and

n = 0 to 50, preferably 0 to 10, most preferably 0 to 5.

The section of the molecule attached to the singly bonded sulfoxy oxygen (but not M) is what is considered the hydro acid (if cleaved between the carboxylate oxygen and the al group) or hydroxy acid ester portion (if not cleaved) of t molecule.

The following compounds are illustrative surface active or self organizer molecules within the present invention. It also to be understood that these molecules are salts or partly formed salts:

decyl 2-(hydrogen sulfooxy)acetate
dodecyl 2-(hydrogen sulfooxy)acetate
tetradecyl 2-(hydrogen sulfooxy)acetate
hexadecyl 2-(hydrogen sulfooxy)acetate
octadecyl 2-(hydrogen sulfooxy)acetate
docosyl 2-(hydrogen sulfooxy)acetate
butyl 2-(hydrogen sulfooxy)acetate
hexyl 2-(hydrogen sulfooxy)acetate
octyl 2-(hydrogen sulfooxy)acetate
nonyl 2-(hydrogen sulfooxy)acetate
tetracosyl 2-(hydrogen sulfooxy)acetate
2-ethylhexyl 2-(hydrogen sulfooxy)acetate
2-ethyldecyl 2-(hydrogen sulfooxy)acetate
2-ethyldodecyl 2-(hydrogen sulfooxy)acetate
2-propyldecyl 2-(hydrogen sulfooxy)acetate
2-butyldecyl 2-(hydrogen sulfooxy)acetate
2-octyldodecyl 2-(hydrogen sulfooxy)acetate
2-dodecyl hexadecyl 2-(hydrogen sulfooxy)acetate
2-tetradecyloctadecyl 2-(hydrogen sulfooxy)acetate
2-ethyldecyl 2-(hydrogen sulfooxy)acetate
hexenyl 2-(hydrogen sulfooxy)acetate
decenyl 2-(hydrogen sulfooxy)acetate
dodecenyl 2-(hydrogen sulfooxy)acetate
tetradecenyl 2-(hydrogen sulfooxy)acetate
hexadecenyl 2-(hydrogen sulfooxy)acetate
octadecenyl 2-(hydrogen sulfooxy)acetate
docosenyl 2-(hydrogen sulfooxy)acetate
tetracosenyl 2-(hydrogen sulfooxy)acetate
nonylphenyl 2-(hydrogen sulfooxy)acetate
decylphenyl 2-(hydrogen sulfooxy)acetate
dodecylphenyl 2-(hydrogen sulfooxy)acetate
tetradecylphenyl 2-(hydrogen sulfooxy)acetate
2-(2-ethoxyethoxy)ethyl 2-(hydrogen sulfooxy)acetate
2-(2-butoxyethoxy)ethyl 2-(hydrogen sulfooxy)acetate
fluorodecyl 2-(hydrogen sulfooxy)acetate
trifluorooctyl 2-(hydrogen sulfooxy)acetate
pentadecafluorodecyl 2-(hydrogen sulfooxy)acetate
fluorododecyl 2-(dihydrogen phosphoxy)acetate
2-(N,N-ditallow-N-methylammonium)ethyl 2-(hydrogen sulfooxy)acetate
3-(N,N-ditallow-N-methylammonium)propyl 2-(hydrogen sulfooxy)acetate
2-(N-nonyl-N,N-dimethylammonium)ethyl 2-(hydrogen sulfooxy)acetate
2-(N-sterayl-N,N-dimethylammonium)ethyl 2-(hydrogen sulfooxy)acetate
2-(N-dodecyl-N,N-dimethylammonium)ethyl 2-(hydrogen sulfooxy)acetate
pentyl 2-(hydrogen sulfooxy)propionate
hexyl 2-(hydrogen sulfooxy)propionate
octyl 2-(hydrogen sulfooxy)propionate
nonyl 2-(hydrogen sulfooxy)propionate
decyl 2-(hydrogen sulfooxy)propionate
dodecyl 2-(hydrogen sulfooxy)propionate
tetradecyl 2-(hydrogen sulfooxy)propionate
hexadecyl 2-(hydrogen sulfooxy)propionate
octadecyl 2-(hydrogen sulfooxy)propionate
docosyl 2-(hydrogen sulfooxy)propionate
tetracosyl 2-(hydrogen sulfooxy)propionate
2-ethylhexyl 2-(hydrogen sulfooxy)propionate
2-ethyldecyl 2-(hydrogen sulfooxy)propionate
2-ethyldodecyl 2-(hydrogen sulfooxy)propionate
2-propyldecyl 2-(hydrogen sulfooxy)propionate

2-butyldoecyl 2-(hydrogen sulfooxy)propionate
2-octyldodecyl 2-(hydrogen sulfooxy)propionate
2-dodecyl hexadecyl 2-(hydrogen sulfooxy)propionate
2-tetradecyloctadecyl 2-(hydrogen sulfooxy)propionate
2-ethyldecyl 2-(hydrogen sulfooxy)propionate
hexenyl 2-(hydrogen sulfooxy)propionate
decenyl 2-(hydrogen sulfooxy)propionate
dodecenyl 2-(hydrogen sulfooxy)propionate
tetradecenyl 2-(hydrogen sulfooxy)propionate
hexadecenyl 2-(hydrogen sulfooxy)propionate
octadecenyl 2-(hydrogen sulfooxy)propionate
docosenyl 2-(hydrogen sulfooxy)propionate
tetracosenyl 2-(hydrogen sulfooxy)propionate
nonylphenyl 2-(hydrogen sulfooxy)propionate
decylphenyl 2-(hydrogen sulfooxy)propionate
dodecylphenyl 2-(hydrogen sulfooxy)propionate
tetradecylphenyl 2-(hydrogen sulfooxy)propionate
2-(2-ethoxyethoxy)ethyl 2-(hydrogen sulfooxy)propionate
2-(2-butoxyethoxy)ethyl 2-(hydrogen sulfooxy)propionate
fluorodecyl 2-(hydrogen sulfooxy)propionate
trlfluorooctyl 2-(hydrogen sulfooxy)propionate
pentadecafluorodecyl 2-(hydrogen sulfooxy)propionate
fluorododecyl 2-(hydrogen sulfooxy)propionate
2-(N,N-ditallow-N-methylammonium)ethyl 2-(hydrogen sulfooxy)propionate
3-(N,N-ditallow-N-methylammonium)propyl 2-(hydrogen sulfooxy)propionate
2-(N-nonyl-N,N-dimethylammonium)ethyl 2-(hydrogen sulfooxy)propionate
2-(N-sterayl-N,N-dimethylammonium)ethyl 2-(hydrogen sulfooxy)propionate
2-(N-dodecyl-N,N-dimethylammonium)ethyl 2-(hydrogen sulfooxy)propionate
hexyl 3-(hydrogen sulfooxy)propionate
octyl 3-(hydrogen sulfooxy)propionate
nonyl 3-(hydrogen sulfooxy)propionate
decyl 3-(hydrogen sulfooxy)propionate
dodecyl 3-(hydrogen sulfooxy)propionate
tetradecyl 3-(hydrogen sulfooxy)propionate
hexadecyl 3-(hydrogen sulfooxy)propionate
octadecyl 3-(hydrogen sulfooxy)propionate
docosyl 3-(dihydrogen phosphoxy)propionate
tetracosyl 3-(hydrogen sulfooxy)propionate
2-ethylhexyl 3-(hydrogen sulfooxy)propionate
2-ethyldecyl 3-(hydrogen sulfooxy)propionate
2-ethyldodecyl 3-(hydrogen sulfooxy)propionate
2-propyldecyl 3-(hydrogen sulfooxy)propionate
2-butyldoecyl 3-(hydrogen sulfooxy)propionate
2-octyldodecyl 3-(hydrogen sulfooxy)propionate
2-dodecyl hexadecyl 3-(hydrogen sulfooxy)propionate
2-tetradecyloctadecyl 3-(hydrogen sulfooxy)propionate
tetradecylphenyl 3-(hydrogen sulfooxy)butyrate
2-(2-ethoxyethoxy)ethyl 3-(hydrogen sulfooxy)butyrate
2-(2-butoxyethoxy)ethyl 3-(hydrogen sulfooxy)butyrate
fluorodecyl 3-(hydrogen sulfooxy)butyrate
trifluorooctyl 3-(hydrogen sulfooxy)butyrate
pentadecafluorodecyl 3-(hydrogen sulfooxy)butyrate
fluorododecyl 3-(hydrogen sulfooxy)butyrate
2-(N,N-ditallow-N-methylammonium)ethyl 3-(hydrogen sulfooxy)butyrate
3-(N,N-ditallow-N-methylammonium)propyl 3-(hydrogen sulfooxy)butyrate
2-(N-nonyl-N,N-dimethylammonium)ethyl 3-(hydrogen sulfooxy)butyrate
2-(N-sterayl-N,N-dimethylammonium)ethyl 3-(hydrogen sulfooxy)butyrate
2-(N-dodecyl-N,N-dimethylammonium)ethyl 3-(hydrogen sulfooxy)butyrate

hexyl 2-(hydrogen sulfooxy)hexanoate
octyl 2-(hydrogen sulfooxy)hexanoate
nonyl 2-(hydrogen sulfooxy)hexanoate
decyl 2-(hydrogen sulfooxy)hexanoate
dodecyl 2-(hydrogen sulfooxy)hexanoate
tetradecyl 2-(hydrogen sulfooxy)hexanoate
hexadecyl 2-(hydrogen sulfooxy)hexanoate
octadecyl 2-(hydrogen sulfooxy)hexanoate
docosyl 2-(hydrogen sulfooxy)hexanoate
tetracosyl 2-(hydrogen sulfooxy)hexanoate
2-ethylhexyl 2-(hydrogen sulfooxy)hexanoate
2-ethyldecyl 2-(hydrogen sulfooxy)hexanoate
2-ethyldodecyl 2-(hydrogen sulfooxy)hexanoate
2-propyldecyl 2-(hydrogen sulfooxy)hexanoate
2-butyldoecyl 2-(hydrogen sulfooxy)hexanoate
2-octyldodecyl 2-(hydrogen sulfooxy)hexanoate
2-dodecyl hexadecyl 2-(hydrogen sulfooxy)hexanoate
2-tetradecyloctadecyl 2-(hydrogen sulfooxy)hexanoate
2-ethyldecyl 2-(hydrogen sulfooxy)hexanoate
hexenyl 2-(hydrogen sulfooxy)hexanoate
decenyl 2-(hydrogen sulfooxy)hexanoate
dodecenyl 2-(hydrogen sulfooxy)hexanoate
tetradecenyl 2-(hydrogen sulfooxy)hexanoate
hexadecenyl 2-(hydrogen sulfooxy)hexanoate
octadecenyl 2-(hydrogen sulfooxy)hexanoate
docosenyl 2-(hydrogen sulfooxy)hexanoate
tetracosenyl 2-(hydrogen sulfooxy)hexanoate
nonylphenyl 2-(hydrogen sulfooxy)hexanoate
decylphenyl 2-(hydrogen sulfooxy)hexanoate
dodecylphenyl 2-(hydrogen sulfooxy)hexanoate
tetradecylphenyl 2-(hydrogen sulfooxy)hexanoate
2-(2-ethoxyethoxy)ethyl 2-(hydrogen sulfooxy)hexanoate
2-(2-butoxyethoxy)ethyl 2-(hydrogen sulfooxy)hexanoate
fluorodecyl 2-(hydrogen sulfooxy)hexanoate
trifluorooctyl 2-(hydrogen sulfooxy)hexanoate
pentadecafluorodecyl 2-(hydrogen sulfooxy)hexanoate
fluorododecyl 2-(hydrogen sulfooxy)hexanoate
2-(N,N-ditallow-N-methylammonium)ethyl 2-(hydrogen sulfooxy)hexanoate
3-(N,N-ditallow-N-methylammonium)propyl 2-(hydrogen sulfooxy)hexanoate
2-(N-nonyl-N,N-dimethylammonium)ethyl 2-(hydrogen sulfooxy)hexanoate
2-(N-sterayl-N,N-dimethylammonium)ethyl 2-(hydrogen sulfooxy)hexanoate
2-(N-dodecyl-N,N-dimethylanmonium)ethyl 2-(hydrogen sulfooxy)hexanoate
2-ethyldecyl 3-(hydrogen sulfooxy)propionate
hexenyl 3-(hydrogen sulfooxy)propionate
decenyl 3-(hydrogen sulfooxy)propionate
dodecenyl 3-(hydrogen sulfooxy)propionate
tetradecenyl 3-(hydrogen sulfooxy)propionate
hexadecenyl 3-(hydrogen sulfooxy)propionate
octadecenyl 3-(hydrogen sulfooxy)propionate
docosenyl 3-(hydrogen sulfooxy)propionate
tetracosenyl 3-(hydrogen sulfooxy)propionate
nonylphenyl 3-(hydrogen sulfooxy)propionate
decylphenyl 3-(hydrogen sulfooxy)propionate
dodecylphenyl 3-(hydrogen sulfooxy)propionate
tetradecylphenyl 3-(hydrogen sulfooxy)propionate
2-(2-ethoxyethoxy)ethyl 3-(hydrogen sulfooxy)propionate
2-(2-butoxyethoxy)ethyl 3-(hydrogen sulfooxy)propionate
fluorodecyl 3-(hydrogen sulfooxy)propionate

trifluorooctyl 3-(hydrogen sulfooxy)propionate
pentadecafluorodecyl 3-(dihydrogen phosphoxy)propionate
fluorododecyl 3-(hydrogen sulfooxy)propionate
2-(N,N-ditallow-N-methylammonium)ethyl 3-(hydrogen sulfooxy)propionate
3-(N,N-ditallow-N-methylammonium)propyl 3-(hydrogen sulfooxy)propionate
2-(N-nonyl-N,N-dimethylammonium)ethyl 3-(hydrogen sulfooxy)propionate
2-(N-sterayl-N,N-dimethylammonium)ethyl 3-(hydrogen sulfooxy)propionate
2-(N-dodecyl-N,N-dimethylammonium)ethyl 3-(hydrogen sulfooxy)propionate
hexyl 3-(hydrogen sulfooxy)butyrate
octyl 3-(hydrogen sulfooxy)butyrate
nonyl 3-(hydrogen sulfooxy)butyrate
decyl 3-(hydrogen sulfooxy)butyrate
dodecyl 3-(hydrogen sulfooxy)butyrate
tetradecyl 3-(hydrogen sulfooxy)butyrate
hexadecyl 3-(hydrogen sulfooxy)butyrate
octadecyl 3-(hydrogen sulfooxy)butyrate
docosyl 3-(hydrogen sulfooxy)butyrate
tetracosyl 3-(hydrogen sulfooxy)butyrate
2-ethylhexyl 3-(hydrogen sulfooxy)butyrate
2-ethyldecyl 3-(hydrogen sulfooxy)butyrate
2-ethyldodecyl 3-(hydrogen sulfooxy)butyrate
2-propyldecyl 3-(hydrogen sulfooxy)butyrate
2-butyldoecyl 3-(hydrogen sulfooxy)butyrate
2-octyldodecyl 3-(hydrogen sulfooxy)butyrate
2-dodecyl hexadecyl 3-(hydrogen sulfooxy)butyrate
2-tetradecyloctadecyl 3-(hydrogen sulfooxy)butyrate
2-ethyldecyl 3-(hydrogen sulfooxy)butyrate
hexenyl 3-(hydrogen sulfooxy)butyrate
decenyl 3-(hydrogen sulfooxy)butyrate
dodecenyl 3-(hydrogen sulfooxy)butyrate
tetradecenyl 3-(hydrogen sulfooxy)butyrate
hexadecenyl 3-(hydrogen sulfooxy)butyrate
octadecenyl 3-(hydrogen sulfooxy)butyrate
docosenyl 3-(hydrogen sulfooxy)butyrate
tetracosenyl 3-(hydrogen sulfooxy)butyrate
nonylphenyl 3-(hydrogen sulfooxy)butyrate
decylphenyl 3-(hydrogen sulfooxy)butyrate
dodecylphenyl 3-(hydrogen sulfooxy)butyrate
hexyl 2-(hydrogen sulfooxy)octanoate
octyl 2-(hydrogen sulfooxy)octanoate
nonyl 2-(hydrogen sulfooxy)octanoate
decyl 2-(hydrogen sulfooxy)octanoate
dodecyl 2-(hydrogen sulfooxy)octanoate
tetradecyl 2-(hydrogen sulfooxy)octanoate
hexadecyl 2-(hydrogen sulfooxy)octanoate
octadecyl 2-(hydrogen sulfooxy)octanoate
docosyl 2-(hydrogen sulfooxy)octanoate
tetracosyl 2-(hydrogen sulfooxy)octanoate
2-ethylhexyl 2-(hydrogen sulfooxy)octanoate
2-ethyldecyl 2-(hydrogen sulfooxy)octanoate
2-ethyldodecyl 2-(hydrogen sulfooxy)octanoate
2-propyldecyl 2-(hydrogen sulfooxy)octanoate
2-butyldoecyl 2-(hydrogen sulfooxy)octanoate
2-octyldodecyl 2-(hydrogen sulfooxy)octanoate
2-dodecyl hexadecyl 2-(hydrogen sulfooxy)octanoate
2-tetradecyloctadecyl 2-(hydrogen sulfooxy)octanoate
2-ethyldecyl 2-(hydrogen sulfooxy)octanoate
hexenyl 2-(hydrogen sulfooxy)octanoate

decenyl 2-(hydrogen sulfooxy)octanoate
dodecenyl 2-(hydrogen sulfooxy)octanoate
tetradecenyl 2-(hydrogen sulfooxy)octanoate
hexadecenyl 2-(hydrogen sulfooxy)octanoate
octadecenyl 2-(hydrogen sulfooxy)octanoate
docosenyl 2-(hydrogen sulfooxy)octanoate
tetracosenyl 2-(hydrogen sulfooxy)octanoate
nonylphenyl 2-(hydrogen sulfooxy)octanoate
decylphenyl 2-(hydrogen sulfooxy)octanoate
dodecylphenyl 2-(hydrogen sulfooxy)octanoate
tetradecylphenyl 2-(hydrogen sulfooxy)octanoate
2-(2-ethoxyethoxy)ethyl 2-(hydrogen sulfooxy)octanoate
2-(2-butoxyethoxy)ethyl 2-(hydrogen sulfooxy)octanoate
fluorodecyl 2-(hydrogen sulfooxy)octanoate
trifluorooctyl 2-(hydrogen sulfooxy)octanoate
pentadecafluorodecyl 2-(hydrogen sulfooxy)octanoate
fluorododecyl 2-(hydrogen sulfooxy)octanoate
2-(N,N-ditallow-N-methylammonium)ethyl 2-(hydrogen sulfooxy)octanoate
3-(N,N-ditallow-N-methylammonium)propyl 2-(hydrogen sulfooxy)octanoate
2-(N-nonyl-N,N-dimethylammonium)ethyl 2-(hydrogen sulfooxy)octanoate
2-(N-sterayl-N,N-dimethylammonium)ethyl 2-(hydrogen sulfooxy)octanoate
2-(N-dodecyl-N,N-dimethylammonium)ethyl 2-(hydrogen sulfooxy)octanoate
didecyl 2-(hydrogen sulfooxy)succinate
dioctyl 2-(hydrogen sulfooxy)succinate
didodecyl 2-(hydrogen sulfooxy)succinate
monodecyl 2-(hydrogen sulfooxy)-3-hydroxy succinate
monododecyl 2-(hydrogen sulfooxy)-3-hydroxy succinate
monotetradecyl 2-(hydrogen sulfooxy)-3-hydroxy succinate
monodecyl 2-(hydrogen sulfooxy)succinate
monododecyl 2-(hydrogen sulfooxy)succinate
monotetradecyl 2-(hydrogen sulfooxy)succinate
monohexadecyl 2-(hydrogen sulfooxy)succinate
hexadecenyl 2-(hydrogen sulfooxy)succinate
octadecenyl 2-(hydrogen sulfooxy)succinate
tetradecenyl 2-(hydrogen sulfooxy)succinate
dodecenyl 2-(hydrogen sulfooxy)succinate
monodecyl 3-(hydrogen sulfooxy)citrate
monododecyl 3-(hydrogen sulfooxy)citrate
monotetradecyl 3-(hydrogen sulfooxy)citrate
monohexadecyl 3-(hydrogen sulfooxy)citrate
monooctadecyl 3-(hydrogen sulfooxy)citrate
didecyl 3-(hydrogen sulfooxy)citrate
didodecyl 3-(hydrogen sulfooxy)citrate
ditetradecyl 3-(hydrogen sulfooxy)citrate

The chemistry of sulfoxy ester compounds is known to give mono-, di-, and tri-substituted sulfoxy ester surfactants. The molecules described in this invention can contain a mixture of such, and preferably the mono-substituted sulfoxy esters and disubstituted sulfoxy esters can be used singly or in combination.

While not wishing to be bound by theory, it is believed that enzymes naturally present in the skin will break down molecules of Formula I or that the molecules will be naturally hydrolyzed upon contact with the skin according to the following scheme:

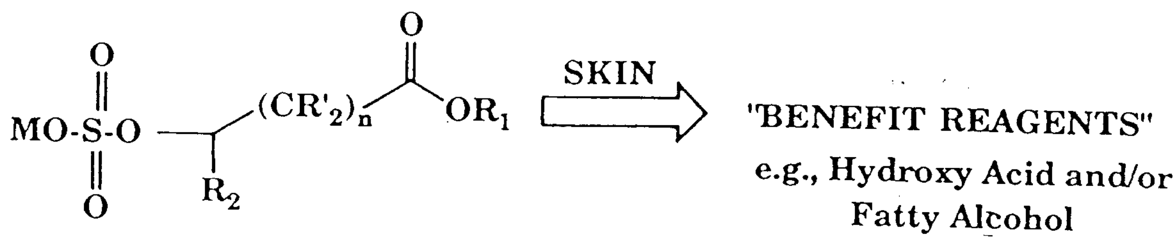

Compositions

Personal product compositions represent further embodiment of the invention and may be, for example, soap bar compositions, facial or body cleansing compositions, shamp for hair or body, conditioners, cosmetic compositions or dental compositions.

In one embodiment of the invention, the surfactant of the invention may be used, for example, in a toilet bar formulation.

Typical soap bar compositions are those comprising fatty a soaps used in combination with a detergent other than fatt acid soap and free fatty acids. Mildness improving salts, such as alkali metal salt or isethionate, are also typical added. In addition other ingredients, such as germicides, perfumes, colorants, pigments, suds-boosting salts and anti-mushing agents may also be added.

Fatty acid soaps are typically alkali metal or alkanol ammonium salts of aliphatic alkane or alkene monocarboxyli acids. Sodium, potassium, mono-, di- and tri-ethanol ammonium cations, or combinations thereof, are suitable fo purposes of the invention. The soaps are well known alkal metal salts of natural or synthetic aliphatic (alkanoic or alkenoic) acids having about 8 to 22 carbons, preferably 1 to about 18 carbons. They may be described as alkali meta carboxylates of acrylic hydrocarbons having about 12 to 22 carbons.

Examples of soap which may be used may be found in U.S. Patent No. 4,695,395 to Caswell et al. and U.S. Patent No. 4,260,507 (Barrett), both of which are incorporated herein reference.

Fatty acid soaps will generally comprise greater than 25% the composition, generally from 30-98%. Preferably, the amount of soap will range from 40% to 70% by weight of the composition.

The compositions will also generally comprise a non-soap detergent which is generally chosen from anionic, nonionic cationic, zwitterionic or amphoteric synthetic detergent materials or mixtures thereof. These surfactants are all well known in the art and are described, for example, in U Patent Nos. 4,695,395 and 4,260,507 discussed above. Thes non-soap actives may comprise from 0 to 50% of the composition.

A certain amount of free fatty acids of 8 to 22 carbons ar also desirably incorporated into soap compositions to act superfatting agents or as skin feel and creaminess enhance If present, the free fatty acids comprise between 1 and 15 of the compositions.

A preferred mildness improving salt which may be added to soap compositions is a simple unsubstituted sodium isethionate. This may be present as 0.1 to 50% of the composition, preferably .5% to 25%, more preferably 2% to about 15% by weight. Other mildness co-actives which may used include betain compounds or ether sulphates. These a may be present at 0.1 to 50% of the composition, preferabl 0.5% to 25%.

The sulfate ester surfactant of the invention may comprise .01 to 45% by weight of the composition (as the monoester) preferably 25% to 40%, and .01% to 10% of the composition the diester), preferably .01% to 5%.

Other optional ingredients which may be present in soap ba compositions are moisturizers such as glycerin, propylene glycol, sorbitol, polyethylene glycol, ethoxylated or methoxylated ether of methyl glucose etc; water-soluble polymers such as collagens, modified cellulases (such as Polymer JR$^{(R)}$), guar gums and polyacrylates; sequestering agents such as citrate, and emollients such as silicones o mineral oil. Another useful set of ingredients are variou cosurfactants and non-soap detergents.

In a further embodiment of the invention, the surfactant o the invention may be present in a facial or body cleansing composition. Examples of such cleaning compositions are described, for example, in U.S. Patent No. 4,812,253 to Sm et al. and U.S. Patent No. 4,526,710 to Fujisawa, both of which are hereby incorporated by reference.

Typically, cleansing compositions will comprise a fatty ac soap together with a non-soap surfactant, preferably a mil synthetic surfactant. Cleaning compositions will also generally include a moisturizer or

emollient and polymeric skin feel and mildness aids. The compositions may further optionally include thickener, conditioners, water soluble polymers, dyes, hydrotropes brighteners, perfumes and germicides.

The fatty acid soaps used are such as those described abov in uses in detergent bar formulations. These soaps are typically alkali metal or alkanol ammonium salts of alipha or alkene monocarboxylic salts. Sodium, potassium, mono-, and triethanol ammonium cations, or combinations thereof a suitable. Preferred soaps are 8 to 24 carbon half acid sa of, for example, triethanolamine.

Surfactants can be chosen from anionic, nonionic, cationic zwitterionic or amphoteric materials or mixtures thereof s as are described in U.S. Patent No. 4,695,395 mentioned above, or in U.S. Patent No. 4,854,333 to Inman et al, her incorporated by reference.

Moisturizers are included to provide skin conditioning benefits and improve mildness. This term is often used as synonymous with emollient and is then used to describe a material which imparts a smooth and soft feeling to skin surface.

There are two ways of reducing water loss from the stratum corneum. One is to deposit on the surface of the skin an occlusive layer which reduces the rate of evaporation. Th second method is to add nonocclusive hydgroscopic substanc to the stratum corneum which will retain water, and make t water available to the stratum corneum to alter its physic properties and produce a cosmetically desirable effect. Nonocclusive moisturizers also function by improving the lubricity of the skin.

Both occlusive and nonocclusive moisturizers can work in t present invention. Some examples of moisturizers are long chain fatty acids, liquid water-soluble polyols, glycerin, propylene glycol, sorbitol, polyethylene glycol, ethoxylated/propoxylated ethers of methyl glucose (eg., methyl gluceth-20) and ethoxylated/-propoxylated ethers of lanolin alcohol (e.g., Solulan-75).

Preferred moisturizers are coco and tallow fatty acids. S other preferred moisturizers are the nonoclusive liquid wa soluble polyols and the essential amino acid compounds fou naturally in the skin.

Other preferred nonocclusive moisturizers are compounds fo to be naturally occurring in the stratum corneum of the sk such as sodium pyrrolidone carboxylic acid, lactic acid, urea, L-proline, guanidine and pyrrolidone. Examples of other nonocclusive moisturizers include hexadecyl, myristy isodecyl or isopropyl esters of adipic, lactic, oleic, stearic, isostearic, myristic or linoleic acids, as well a many of their corresponding alcohol esters (sodium isostearoyl-2 lactylate, sodium capryl lactylate), hydroly protein and other collagen-derived proteins, aloe vera gel and acetamide MEA.

Some occlusive moisturizers include petrolatum, mineral oi beeswax, silicones, lanolin and oil-soluble lanolin derivatives, saturated and unsaturated fatty alcohols such behenyl alcohol, squalene and squalane, and various animal and vegetable oils such as almond oil, peanut oil, wheat g oil, linseed oil, jojoba oil, oil of apricot pits, walnuts palm nuts, pistachio nuts, sesame seeds, rapeseed, cade oil,corn oil, peach pit oil, poppyseed oil, pine oil, cast oil, soybean oil, avocado oil, safflower oil, coconut oil, hazelnut oil, olive oil, grape seed oil and sunflower seed oil.

Other examples of both types of moisturizers are disclosed "Emollients -- A Critical Evaluation," by J. Mausner, Cosmetics & Toiletries, May 1981, incorporated herein by reference.

The polymeric skin feel and mildness aids useful in the present invention are the cationic, anionic, amphoteric, a the nonionic polymers used in the cosmetic field. Reduced skin irritation benefits as measured by patch testing of cationic and nonionic types of polymers are set out in "Polymer JR for Skin Care" Bulletin, by Union Carbide, 197 The cationics are preferred over the others because they provide better skin feel benefits.

The amount of polymeric skin feel and mildness aids found useful in the composition of the present invention is from about 0.01% to about 5%, preferably from about 0.3% to abo 4%. In bar compositions with less than 5.5% soap, the polymer is used at a level of 2% to 5%, preferably 3% or more.

Other types of high molecular weight polymeric skin feel a skin mildness aids, such as nonionic guar gums, Merquats 1 and 550, made by Merck & Co, Inc.; JAGUAR C-14-S made by Stein Hall; Mirapol A15 made by Miranol Chemical Company, Inc.; and Galactasol 811, made by Henkel, Inc.; plus other are usable. The polymer also provides enhanced creamy lat benefits.

The nonionic polymers found to be useful include the nonio polysaccharides, e.g., nonionic hydroxypropyl guar gums, offered by Celanese Corp. A preferred nonionic hydroxypro guar gum material is JAGUAR$^R$ HP-60 having molar substituti of about 0.6. Another class of useful nonionics is the cellulosic nonionic polymers, e.g., HEC and CMC.

The cationic polymers employed in this invention also prov a desirable silky, soft, smooth in-use feeling. The preferred level for this invention is 0.1-5% of the composition. There is reason to believe that the positive charged cationic polymers can bind with negatively charges sites on the skin to provide a soft skin feel after use. to be bound by any theory, it is believed that the greater the charge density of the cationic polymer, the more effective it is for skin feel benefits.

Other suitable cationic polymers are copolymers of dimethylaminoethylmethacrylate and acrylamide and copolyme of dimethyldiallylammonium chloride and acrylamide in whic the ratio of the cationic to neutral monomer units has bee selected to give a copolymer having a cationic charge. Ye other suitable types of cationic polymers are the cationic starches, e.g., Sta-Lok$^R$300 and 400 made by Staley, Inc.

A more complete list of cationic polymers useful in the present invention is described in U.S. Pat. No. 4,438,095, Grollier/Allec, issued Mar. 20, 1984, incorporated herein reference. Some of the more preferred cationics are liste in Col. 3, Section 2; Col. 5, section 8; Col. 8, section 1 and Col. 9, lines 10-15 of the Grollier/Allec patent, incorporated herein by reference.

In a further embodiment of the invention, the surfactant o the invention may be used, for example, in a bar or body shampoo. Examples of such compositions are described in U Patent No. 4,854,333, to Inman and U.S. Patent No. 4,526,7 to Fujisawa, both of which are hereby incorporated by reference.

The shampoo compositions which may be used typically compr a surfactant selected from any one of a wide variety of surfactants known in the art (such as those described in U Patent No. 4,854,333, incorporated herein by reference). shampoo compositions may additionally comprise a compound considered useful for treating dandruff, e.g. selenium sulfide.

The compositions all may also optionally comprise a suspending agent, for example, any of several acyl derivat materials or mixtures thereof. Among these are ethylene glycol esters of fatty acids having 16 to 22 carbons. Preferred suspending agents include ethylene glycol stearates, both mono- and distearate. Preferred alkanol amides are stearic monoethanolamide, stearic diethanolamide and stearic monoisopropanolamide. Still other long chain acyl derivatives include long chain esters of long chain fatty acids (e.g., stearyl stearate, cetyl palmitate), glyceryl esters (e.g. glyceryl distearate), and long chain esters of long chain alkanol amides (e.g., stearamide DEA distearate, stearamide MEA stearate).

Still other suitable suspending agents are alkyl (16 to 22 carbon) dimethyl amine oxides, such as stearyl dimethyl amine oxide. If the compositions contain an amine oxide or a long chain acyl derivative as a surfactant, these components may also provide the suspending function and additional suspending agent may not be needed.

Xanthan gum is another aspect used to suspend, for example, selenium sulfide which may be in the present compositions. This biosynthetic gum material is commercially available and is a heteropolysaccharide with a molecular weight of greater than l million. It is believed to contain D-glucose, D-mannose and D-glucuronate in the molar ratio of 2.8:2.0:2.0. The polysaccharide is partially acetylated with 4.7% acetyl. Supplemental information on these agents is found in Whistler, Roy L. (Editor), Industrial Gums --Polysaccharides and Their Derivatives New York: Academic Press, 1973. Kelco, a Division of Merck & Co., Inc., offers xanthan gum as KeltrolR.

A particularly preferred suspending system comprises a mixture of xanthan gum, present at a level of from about 0.05% to about 1.0%, preferably from about 0.2% to about 0.4%, of the compositions, together with magnesium aluminum silicate ($Al_2Mg_8Si_2$), present at a level of from about 0.1% to about 3.0%, preferably from about 0.5% to about 2.0%, of the compositions. Magnesium aluminum silicate occurs naturally in such smectite minerals as colerainite, saponite and sapphire. Refined magnesium aluminum silicates useful herein are readily available, for example as veegum, manufactured by R.T. Vanderbilt Company, Inc. Mixtures of suspending agents are also suitable for use in the compositions of this invention.

Other useful thickening agents are the cross-linked polyacrylates such as those manufactured by B. F. Goodrich and sold under the Carbopol$^{(R)}$ tradename.

Another optional component for use in the present compositions is an amide. The amide used in the present compositions can be any of the alkanolamides of fatty acids known for use in shampoos. These are generally mono- and diethanolamides of fatty acids having from about 8 to 24 carbon atoms. Preferred are coconut monoethanolamide, lauric diethanolamide and mixtures thereof. The amide is present at a level of from about 1% to about 10% of the compositions.

The compositions may also contain nonionic polymer material which is used at a low level to aid in dispersing particles. The material can be any of a large variety of types including cellulosic materials such as hydroxypropyl methyl cellulose, carboxymethyl cellulose, hydroxyethyl cellulose and sodium carboxymethyl cellulose as well as mixtures of these materials. Other materials include alginates, polyacrylic acids, polyethylene glycol and starches, among many others. The nonionic polymers are discussed in detail in Industrial Gums, edited by Roy L. Whistler, Academic press, Inc., 1973, and Handbook of Water-Soluble Gums and Resins, edited by Robert L. Davidson, McGraw-Hill, Inc., 1980. Both of these books in their entirety are incorporated herein by reference.

When included, the nonionic polymer is used at a level of from about 0.001% to about 0.1%, preferably from about 0.0 to about 0.05%, of the composition. Hydroxypropyl methyl cellulose is the preferred polymer.

Another suitable optional component useful in the present compositions is a nonvolatile silicone fluid.

The nonvolatile silicone fluid may be either a polyalkyl siloxane, a polyaryl siloxane, a polyalkylarly siloxane or polyether siloxane copolymer and is present at a level of from about 0.1% to about 10.0%, preferably from about 0.5% about 5.0%. Mixtures of these fluids may also be used and are preferred in certain executions. The dispersed silico particles should also be insoluble in the shampoo matrix. This is the meaning of "insoluble" as used herein.

The essentially nonvolatile polyalkyl siloxane fluids that may be used include, for example, polydimethyl siloxanes w viscosities ranging from about 5 to about 600,000 centisto at 25° C. These siloxanes are available, for example, fro the General Electric Company as the Viscasil series and fr Dow Corning as the Dow Corning 200 series. The siloxane viscosity can be measured by means of a glass capillary viscometer as set forth in Dow Corning Corporate Test Meth CTM0004, July 20, 1970. Preferably the viscosity of the these siloxanes range from about 350 centistokes to about 100,000 centistokes.

The essentially nonvolatile polyether siloxane copolymer t may be used is, for example, a polypropylene oxide modifie dimethylpolysiloxane (e.g., Dow Corning DC-1248), although ethylene oxide or mixtures of ethylene oxide and propylene oxide may also be used.

Suitable silicone fluids are described in U.S. Pat. No. 2,826,551, Geen; U.S. Pat. No. 3,946,500, June 22, 1976, Drakoff; U.S. Pat. No. 4,364,837, Pader; and British Pate 849,433, Woolston. All of these patents are incorporated herein by reference. Also incorporated herein by referenc is Silicon Compounds, distributed by Petrarch Systems, Inc 1984. This reference provides a very good listing of suitable silicone materials.

Another silicone material useful is silicone gum. Silicon gums are described by Petrarch and others including U.S. P No. 4,152,416, May 1, 1979, Spitzer, et al., and Noll, Chemistry and Technology of Silicones, New York, Academic Press, 1968. Useful silicone gums are also described in General Electric Silicone Rubber Product Data Sheets SE 30 SE 33, SE 54 and SE 76. All of these references are incorporated herein by reference. "Silicone gum" material denote high molecular weight polydiorganosiloxanes having mass molecular weight of from about 200,000 to about 1,000,000. Specific examples include polydimethylsiloxane (polydimethylsiloxane) (methylvinylsiloxane) copolymer, poly(dimethylsiloxane) (diphenyl) (methylvinylsiloxane) copolymer, and mixtures thereof. Mixtures of silicone flu and silicone gums are also useful herein.

The shampoos herein can contain a variety of other nonessential optional components suitable for rendering su compositions more formulatable, or aesthetically and/or cosmetically acceptable. Such conventional optional ingredients are well-known to those skilled in the art and include, e.g., preservatives, such as benzyl alcohol, meth paraben, propyl paraben, and imidazolinidyl urea; cationic surfactants, such as cetyl trimethyl ammonium chloride, lauryl trimethyl ammonium chloride, tricetyl methyl ammoni chloride, stearyldimethyl benzyl ammonium chloride, and di(partially hydrogenated tallow) dimethylammonium chlorid menthol; thickeners and viscosity modifiers, such as block polymers of ethylene oxide and propylene oxide such as Pluronic F88 offered by BASA Wyandotte, sodium chloride, sodium sulfate, propylene glycol, and ethyl alcohol; pH adjusting agents, such as citric acid, succinic acid, phosphoric acid, sodium hydroxide, sodium carbonate; perfumes; dyes; and sequestering agents, such as disodium ethylenediamine tetraacetate. Such agents generally are u individually at a level of from about 0.01% to about 10%, preferably from about 0.5% to about 5.0%, of the compositi

In a further embodiment of the invention, the surfactant o the invention may be used in a conditioner composition suc as is taught and described in U.S. Patent No. 4,913,828 to Caswell et al. which is hereby incorporated by reference.

More particularly, conditioner compositions are those containing a conditioning agent (e.g. alkylamine compounds such as those described in U.S. Patent 4,913,828.

In a fifth embodiment of the invention, the surfactant may used in a cosmetic composition, such as is taught and is describe in EP 0,371,803.

Such compositions generally comprise thickening agents, preservatives and further additions.

The composition may comprise polymer thickener in an amoun sufficient to adjust the viscosity of the composition, so to facilitate dispensing it conveniently onto the body surface.

Examples of polymer thickeners include: anionic cellulose materials, such as sodium carboxy methyl cellulose; anioni polymers such as carboxy vinyl polymers, for example, Carbomer 940 and 941; nonionic cellulose materials, such a methyl cellulose and hydroxy propyl methyl cellulose; cationic cellulose materials, such as Polymer JR 400; cationic gum materials, such as Jaguar C13 S; other gum materials such as gum acacia, gum tragacanth, locust bean gum, guar gum and carrageenan; proteins, such as albumin a protein hydrolysates; and clay materials, such as bentonit hectorite, magnesium aluminum silicate, or sodium magnesiu silicate.

Generally, the thickening agent may comprise from 0.05 to preferably 0.1 to 1% by weight of the composition.

The composition according to the invention can also optionally comprise a preservative to prevent microbial spoilage.

Examples of preservatives include:

(i) Chemical preservatives, such as ethanol, benzoic acid sodium benzoate, sorbic acid, potassium sorbate, sodi propionate and the methyl, ethyl, propyl and butyl esters of p-hydroxybenzoic acid 2-bromo-2-nitropropane-1, 3-diol, phenoxyethanol, dibromodicyanobutane, formalin and Tricolsan. The amount of chemical preservative optionally to be incorporated in the composition according to the invention will generally be from 0.05 to 5%, preferab from 0.01 to 2% by weight, the amount chosen being sufficient to arrest microbial proliferation.

(ii) Water activity depressants, such as glycerol, propyle glycol, sorbitol, sugars and salts, for examples alka metal halides, sulphates and carboxylates. When employing a water activity depressant, sufficient should be incorporated in the composition according t the invention to reduce the water activity ($\alpha_w$) from to <0.9, preferably to <0.85 and most preferably < 0. the lowest of these values being that at which yeasts molds and fungi will not proliferate.

The composition can also contain other optional adjuncts, which are conventionally employed in compositions for topi application to human skin. These adjuncts, when present, will normally form the balance of the composition.

Examples of optional adjuncts include vehicles, the select of which will depend on the required product form of the composition. Typically, the vehicle when present, will be chosen from diluents, dispersants or carriers for the dial or dialkenyl phosphate salt so as to ensure an even distribution of it when applied to the skin.

Compositions according to this invention can include water a vehicle, usually with at least one other cosmetically-acceptable vehicle.

Vehicles other than water that can be used in compositions according to the invention can include liquids or solids a emollients, solvents, humectants, thickeners and powders. Examples of each of these types of vehicles, which can be used singly or as mixtures of one or more vehicles, are as follows:

Emollients, such as stearyl alcohol, glyceryl monolaurate, glyceryl monoricinoleate, glyceryl monostearate, propane-1 2-diol, butane-1.3 diol, docosan-1,2-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cety palmitate, silicone oils such as dimethylpolysiloxane, di-n-butyl sebacate, isopropyl myristate, isopropyl palmitate, isopropyl stearate, butyl stearate, polyethylen glycol, triethylene glycol, lanolin, cocoa butter, corn oi cotton seed oil, tallow, lard, olive oil, palm kernel oil, rapeseed oil, safflower seed oil, soybean oil, sunflower s oil, olive oil, sesame seed oil, coconut oil, arachis oil, castor oil, acetylated lanolin alcohols, petroleum, minera oil, butyl myristate, isostearic acid, palmitic acid, isopropyl linoleate, lauryl lactate, myristyl lactate, dec oleate, myristyl myristate;

Propellants, such as trichlorofluoromethane, dichlorodifluoromethane, dichlorotetrafluoromethane, monochlorodifluoromethane, trichlorotrifluoromethane, propane, butane, isobutane, dimethyl ether, carbon dioxide nitrous oxide;

Solvents, such as ethyl alcohol, methylene chloride, isopropanol, acetone, castor oil, ethylene glycol monoethy ether, diethylene glycol monobutyl ether, diethylene glyco monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starc gums, colloidal silicon dioxide, sodium polyacrylate, tetr alkyl and/or trialkyl aryl ammonium smectites, chemically modified magnesium aluminum silicate, organically modified montmorillonite clay, hydrated aluminum silicate, fumed silica, carboxyvinyl polymer, sodium carboxymethyl cellulo ethylene glycol monostearate.

The cosmetically acceptable vehicle, when present, will usually form from 0.01 to 99.9%, preferably from 59 to 98% weight of the composition, and can, in the absence of othe cosmetic adjuncts, form the balance of the composition.

A wide variety of conventional sunscreening agents, such a those described in U.S. Patent No. 4,919,934 to Deckner et al. hereby incorporated by reference, may also be used in cosmetic compositions of the invention.

Such agents include, for example, p-Aminobenzoic acid, its salts and its derivatives, anthranilates, salicylates, cinnamic acid derivatives, di- and trihydroxy cinnamic aci derivatives, hydrocarbons such as

diphenylbutadiene and stilbene, dibenzalacetone and benzalacetophenone, naphthasulfonates, di-hydroxy naphthloic acid and its salt hydroxy diphenylsulfonates, coumarin derivatives, diazoles quinine salts, quinoline derivatives, hydroxy or methoxy substituted benzophenones, uric or vilouric acid, tannic a and its derivatives, hydroquinone, and benzophenones.

In a further embodiment of the invention, the surfactant m be used in a toothpaste composition such as is taught and described in U.S. Patent No. 4,935,227 to Duckworth, which hereby incorporated by reference.

Such compositions generally comprise abrasive gels (e.g. calcium carbonate), oral therapeutic agents (e.g., flourin containing compound), coactives, flavoring agents, sweeten agents, humectants and binding or thickening gels.

Preferred toothpastes of this invention comprise 0 to 1.5% weight of anionic surfactant. In more preferred products amount of anionic surfactant is 0 to 1% by weight with mos preferred amounts being 0 to 0.75% by weight.

Toothpastes of this invention may include other surfactant especially non-ionic surfactants.

Toothpaste of the invention will also comprise the usual additional ingredients in particular humectant binder or thickening agent.

Humectants which may be used include glycerol, sorbitol syrup, polyethylene glycol, lactitol, xylitol or hydrogena corn syrup. The total amount of humectant present will generally range from 10% to 85% by weight of the toothpast

Numerous binding or thickening agents have been indicated use in toothpastes, preferred ones being sodium carboxymethylcellulose, cross-linked polyacrylates and xanthan gum. Others include natural gum binders such as g tragacanth, gum karaya and gum arabic, Irish moss, alginat and carrageenans. Silica thickening agents include the silica aerogels and various precipitated silicas. Mixture of binders and thickeners may be used. The amount of bind and thickening agent included in a toothpaste is generally between 0.1 and 15% by weight.

In a further embodiment of the invention, the molecule of invention may be used in a light duty liquid detergent composition such as those taught in U.S. Patent No. 4,671, to Lamb et al, hereby incorporated by reference, which pat is also hereby incorporated by reference.

Generally such compositions comprise a mixture of sulphate and sulphonate anionic surfactants together with a suds stabilizing agent. These compositions may also comprise nonionic surfactants designed to reduce the level of non-performing ingredients such as solvents and hydrotrope and zwitterionic surfactants for providing enhanced grease and particulate soil removal performance.

Among other ingredients which may also be used in such compositions are opacifiers (e.g. ethylene glycol distearate), thickeners (e.g., guar gum), antibacterial agents, antitarnish agents, heavy metal chelators (e.g. (ETDA), perfumes and dyes.

In a further embodiment of the invention the molecule of t invention may be used in underarm deodorant/antiperspirant compositions such as those taught in U.S. Patent No. 4,919,934 to Deckner, U.S. Patent No. 4,944,937 to McCall U.S. Patent No. 4,944,938 to Patini, all of which patents hereby incorporated by reference.

Such compositions generally comprise a cosmetic stick (gel wax) composition which in turn generally comprises one or more liquid base materials (e.g., water, fatty acid and fa alcohol esters, water-insoluble ethers and alcohols, polyorganosiloxanes); a solidifying agent for solidifying liquid base; and an active component such as bacteriostats fungistats (for anti-deodorant activity) or astringent metallic salts (for antiperspirant activity).

These compositions may also comprise hardeners, strenghteners, emollients, colorants, perfumes, emulsifier and fillers.

While various compositions are described above, these shou not be understood to be limiting as to what other personal product compositions may be used since other compositions which may be known to those of ordinary skill in the art a also contemplated by this invention.

## PREPARATION OF ESTER SULFATES

The molecules of the invention were obtained essentially through a process in which a desired hydroxy carboxylic ac (or hydroxy acid ester or lactone) was prepared and the molecule thus was then sulfated to obtain the final produc

For example, one hydroxy acid, alkyl lactate, may be prepa by direct esterification or transesterification as taught Dixon et al., J. Am. Chem. Soc. 72: 1918-1922 (1950) and Holtin et al., Verlag Chemie, 232-238 (1971). Preparation alkyl alkanoates is described in further detail in the examples.

The preparation of the hydroxy carboxylic acid or hydroxy carboxylic acid ester (including lactone esters) which is be sulfated does not necessarily result in a pure yield of the hydroxy acid or the hydroxy acid ester and, in fact, unless vacuum distillation is used (which does result in a nearly pure yield), the preparation of the hydroxy acid or the hydroxy acid ester additionally results in the preparation of a free alcohol.

The sulphation of the free alcohol results in the preparat of an alkyl sulfate which, while not a benefit-containing reagent contemplated by the invention, is a useful surfact (such as sodium dodecyl sulfate (SDS) for example). This important because it shows that even the free alcohol by-product of the reaction for preparing hydroxy acid or hydroxy acid ester prior to sulphation can be used to prep a useful, commercial product.

Having obtained the hydroxy acid, the hydroxy acid ester and/or free alcohol (which, as indicated above, can be sulfated to obtain commercial surfactants), sulphation of molecule is achieved as follows:
First, the sulphation temperature may range from about -80 to about 120 °C, preferably at about -20 ° to 35 °C, most preferably about 0-5 °C.

As a sulfating agent, a number of sulfating agents such as are known in the art, e.g., sulfonic acid, chlorosulfonic acid, amidosulfonic acid and sulphur trioxide may be used. $SO_3$/pyridine complex may also be used. Other possible sulfating agents are described, for example, in "Sulphatio and Related Reactions", Gilbert E. Interscience Publisher, 1965, Chap. VI, pp. 339-383. The preferred agent is chlorosulfonic acid or $SO_3$.

When a hydroxy carboxylic acid ester substrate is to be sulfated, it is generally difficult to sulfate such ester linkages (or other weak linkages). Accordingly, in a preferred embodiment of the invention, sulphation is accomplished under anhydrous conditions. In order to minimize hydrolysis of the ester (or other weak bond) link In one preferred embodiment, sulphation is done under a nitrogen blanket of atmosphere.

In general, at least about 0.8 to 4 equivalents, preferabl 0.8 to 2 equivalents, most preferably 0.8 to 1.2 equivalen of sulfating agents are used.

An acid scavenger may also be used to minimize hydrolyzed by-products. If an acid scavenger is used, preferred scavengers which may be used include pyridine or triethylamine.

Other scavengers which may be used to minimize hydrolysis include weak organic bases such as triethanolamine, inorga bases such as sodium carbonate or polymeric bases. The sulphation reaction generally may take from about 5 minutes to 24 hours, preferably 5 minutes to 6 hours, most preferably, 5 minutes to 60 minutes. Preferably the react should run no longer than one hour in order to minimize hydrolysis.

Solvents which may be used (although not required) for the sulphation include tetrahydrofuran (THF), hydrocarbon solvents and halocarbons solvents (e.g., chloroform)
Finally, it is preferred to reduce heat, e.g., with an ice quench, in order to minimize hydrolysis of both carboxy an sulfate esters.

Table 1 below provides a summary of sulphation reaction us various alkyl alkanoates as a starting reactant:

<u>Table 1 - Preparation of Alkyl Sulfooxyalkanoates Sodium Salt</u>

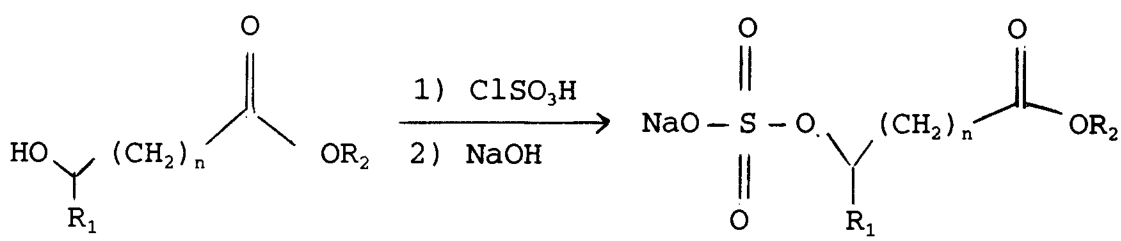

Table 1

| $R_1$ | n | $R_2$ | Crude Yield | Isolated Yield |
|---|---|---|---|---|
| $CH_3$ | 0 | $C_{10}H_{21}$ | Quantitative | 99% |
| $CH_3$ | 0 | $C_{12}H_{25}$ | Quantitative | --- |
| $CH_3$ | 0 | $C_8H_{17}$ | ca.60% | --- |
| $CH_3$ | 1 | $C_{10}H_{21}$ | Quantitative | 97% |
| $CH_3$ | 1 | $C_{12}H_{25}$ | Quantitative | 99% |
| $(CH_2)_5CH_3$ | 0 | $C_{10}H_{21}$ | Quantitative | 62% |
| > 90% Pure by Hyamine Titration <1% of Cl | | | | |

Sulphation results in the formation of a hydrogen sulfooxy acid or the ester thereof (or of an alkyl sulfate if the substrate was a free alcohol by-product of the reaction in which the hydroxy acid and the ester were prepared). The molecule is then neutralized with inorganic or organic bases (e.g., sodium bicarbonate, sodium hydroxide or triethanolamine). At least partial neutralization is required. Preferred bases used for neutralization include sodium and potassium salts and triethanolamine.

Among alkyl sufooxyalkonates produced using these techniques are decyl 2-sulfooxypropionate (DSP), dodecyl 2-sulfooxypropionate (LSP), decyl 3-sulfooxybutyrate (DSB), dodecyl 3-sulfooxybutyrate (LSB), decyl 2-sulfooxyoctanoate (DSO) and octyl-2-sulfooxypropionate (OSP). These molecules are set forth below:

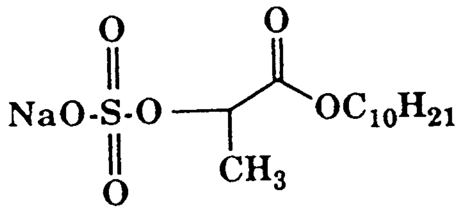

DSP

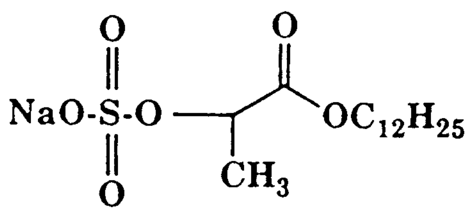

LSP

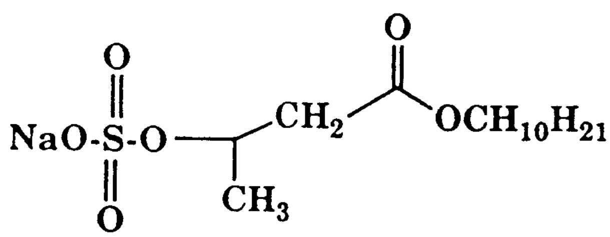

DSB

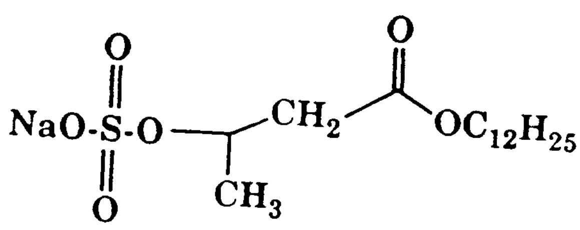

LSB

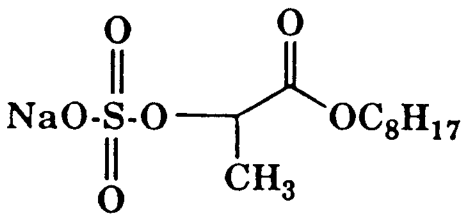

*OSP*

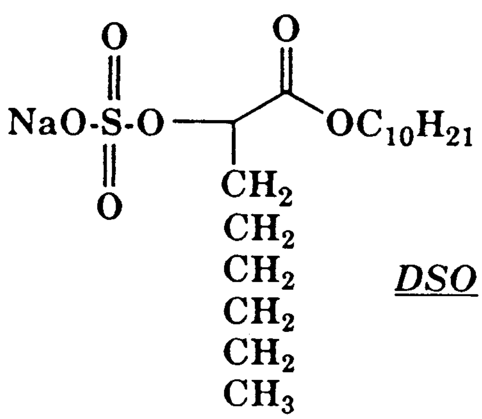

*DSO*

Preparation of various alkanoates is set forth in Table II below.

TABLE II

## PREPARATION OF ALKYL ALKANOATE

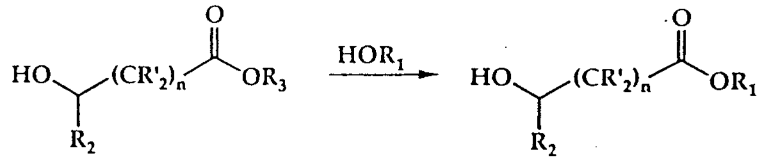


| Entry | $R_2$ | R' | n | $R_3$ | $R_1$ | Dist. Yield | Purity by GC |
|---|---|---|---|---|---|---|---|
| 1 | $CH_3$ | H | 0 | H | $-(CH_2)_7CH_3$ | 36-65% | 96.0% |
| 2 | $CH_3$ | H | 0 | H | $-(CH_2)_9CH_3$ | 12-58% | 95.0% |
| 3 | $CH_3$ | H | 0 | $CH_3$ | $-(CH_2)_7CH_3$ | 44% | 95.0% |
| 4 | $CH_3$ | H | 0 | $CH_3$ | $-(CH_2)_9CH_3$ | 45-62% | >99.0% |
| 5 | $CH_3$ | H | 0 | $CH_3$ | $-(CH_2)_{11}CH_3$ | 62% | 100.0% |
| 6 | $CH_3$ | H | 1 | H | $-(CH_2)_9CH_3$ | 58% | 89.0% |
| 7 | $CH_3$ | H | 1 | H | $-(CH_2)_{11}CH_3$ | 60% | 91.0% |
| 8 | $(CH_3)_2$ | H | 0 | H | $-(CH_2)_9CH_3$ | 65% | 99.5% |
| 9 | $-(CH_2)_3CH_3$ | H | 0 | H | $-(CH_2)_9CH_3$ | 49% | 96.0% |
| 10 | $-(CH_2)_5CH_3$ | H | 0 | H | $-(CH_2)_9CH_3$ | 77% | 99.0% |
| 11 | β-Butylrolactone | | | — | $-(CH_2)_{11}CH_3$ | 89%* | 80.0% |
| 12 | $CH_3$ | H | 0 | H | $-(CH_2)_{13}CH_3$ | 70%* | 69.0% |
| 13 | H | H | 0 | $CH_3$ | $-(CH_2)_{11}CH_3$ | 98%* | 78.0% |

*GC Yield.

As indicated above, free alcohols (e.g., fatty acid alcohols) may be a by-product of the preparation of the alkyl alkanoate (especially if no vacuum distillation is used). These by-products may be sulfated to produce commercial alkyl sulfates such as SDS.

The following examples are intended to illustrate the invention and facilitate its understanding and are not meant to limit the invention in any way.

Example 1

General Procedures and Techniques Used

Boiling points were measured during vacuum distillation an are un-corrected. Proton magnetic resonance ($^1H$ NMR) spec were recorded on a Bruker 200 MHz FT spectrometer or Varia 300 MHz FT spectrometer or Varian T-60 spectrometer. Carb magnetic resonance spectra ($^{13}C$ NMR) were recorded on a Bruker 200 FT (50 MHz) spectrometer. Proton and carbon chemical shifts are reported in parts per million downfiel from tetramethylsilane as an internal standard or other silylated standard. Coupling constants (J value) are give in Hertz (Hz) and spin multiplicities are indicated as follows: s (singlet), d (doublet), t (triplet), q (quarte m (multiplet), or br (broad). The deuterated NMR solvents contain 99.0-99.8% deuterium in the indicated position and these solvents were purchased from Aldrich Chemical Compan and from Cambridge Isotope Laboratories. Infrared spectra (IR) were recorded on a Perkin-Elmer model 298 spectromete or a Nicolet 5SX FT IR spectrometer using a NaCl cell. Fe positions are listed as vs (very strong), s (strong), m (medium), w (weak) or b(broad).

High resolution fast atomic bombardment mass spectra (FAB M.S.) were obtained on a hybrid VG 705 EQ (magnetic sector quadrople hybrid instrument) at Georgia Tech Institute University, Atlanta, Georgia, USA. FAB M.S. were obtained a tandem quadropole Finnigan MAT TSQ70 instrument. Gas chromatography (GC) was performed using a model 5840A purchased from Hewlett Packard with a 5% OV101 methyl sili packed column (80/100 chromosorb 6" x 1/8"). The GC parameters were set as follows: Inj. temp. = 250 ° C, initi column. temp. = 70 ° C, final column. temp. = 250 ° C, rate = 10 ° C/minute.

Chlorosulfonic acid was purchased from Aldrich Chemical Chemical and were used as received. Alcohols (octanol, decanol, dodecanol alcohol, tetradecyl alcohol) were reage grade quality and were used as received. Lactic acid, 3-hydroxy butyric acid; β-butyrolactone and hydroxycaproic acid were received from Aldrich and were used as received. Hydroxyoctanoic acid (HCA) were purchased from Lancaster Synthesis and was used as received.

Example 2

Preparation of Octyl 2-Sulfoxypropionate Sodium Salt

Preparation of Octyl Lactate by Transesterification

A 500 mL one neck round bottom flask equipped with a distillation apparatus and nitrogen inlet/outlet was charg with 100.0 g (0.85 moles) of ethyl lactate, 220.49 g (1.6 moles) of octyl alcohol and 0.42 g of sulphuric acid. The reaction was refluxed for 8 hours and ethanol was collecte as formed. The acid was neutralized by washing three time with 100 mL saturated sodium bicarbonate solution. The organic layer was collected and dried over magnesium sulfa Excess octyl alcohol was removed by high vacuum distillati and three fractional distillations led to 75.0 g (44% yiel of clear colorless oil. According to GC, the product is 9 pure.

B.P. = 93.5-94.5 ° C/0.45 mm (lit B.P. = 87 ° C/1.0 mm)

GC (Rt): 11.8

IR (neat, in $cm^{-1}$): 3420 (br.m), 1737.4 (s), 1480 (m), 1212 (m), 1131 (s) $^1$H NMR (200 MHz FT, $CDCl_3$ with TMS): δ 4.4 (q = 6.8 Hz, 1H), 4.2 (t,J = 6.5 Hz,2H), 3.3 (-OH, br.s, 1H), 1.7 (br.t, J = 6.5 Hz, 2H), 1.4 (d,J = 6.8 Hz, 3H), 1.3 (br.s, 10H), 0.9 (br. t,J = 6.5 Hz, 3H)

$^{13}$C NMR (50 MHz, CDCl3 in ppm): 175.9, 66.8, 65.8, 31.85, 29.3, 29.23, 28.64, 25.87, 22.71, 20.5, 14.1

Preparation of Octyl Lactate by Direct Esterification

A 2 liter one neck round bottom flask equipped with a Dean Stark trap, condenser and nitrogen inlet/outlet was charge with 150.0 g (1.67 moles) lactic acid, 293.0 g (2.25 moles octyl alcohol, 6 ml sulphuric acid and 500 ml toluene. Mixture was heated to 130 ° C for 6 hours and water was collected as the reaction proceeded. The acid was neutralized by washing three times with 50 ml saturated sodium bicarbonate solution. The organic layer was collec and dried over magnesium sulfate. Octyl lactate was distilled under high vacuum to yield 119.9 g (35.6% yield) clear colorless liquid. According to GC the product is 96 pure.

Spectral data identical as described above.

Preparation of Octyl 2-Sulfooxypropionate Sodium Salt from Octyl Lactate

A 50 mL Schlenk flask equipped with a spin bar was charged with 1.73 g (14.9 mmole) of $ClSO_3H$. The reaction flask wa cooled to ca. 0 ° C in an ice water bath under a nitrogen atmosphere, and octyl lactate (2.5 g, 12.4 mmole) was slow added. After addition was completed, the reaction was maintained at 0 ° C and was stirred for 15 minutes. A solut of sodium hydroxide in deionized water/ice was added, and pH was adjusted to ca. 7.0. The water layer was collected and lyophilized to give 4.6 g of a crude white product. T white solid was not further purified. Proton NMR indicate the presence of the hydrolyzed product, lactate (ca. 45%).

IR (nujol, in $cm^{-1}$): 1735 (s), 1255 (br.s), 1220 (br.s), 11 (s), 1060 (s), 1030 (s), 950 (s)

$^1$H NMR (100 MHz FT, $D_20$) δ 4.65 (apparent q, 1H), 4.0 (m, 2H), 1.5 (br.m, 2H), 1.35 (d,J = 6.9 Hz, 3H), 1.15 (br.s, 10H), 0.7 (br.5, 3H)

Low Resolution FAB M.S. (from glycerol/$H_20$, (%) relative intensity): m/z 327 (M + Na)

Example 3

Preparation of Decyl 2-Sulfooxypropionate Sodium Salt

Preparation of Decyl Lactate by Transesterfication

A 250 mL round bottom flask equipped with a spin bar and a distillation column was charged with 100 g (0.96 moles) of methyl lactate, 304.1 g (1.92 moles) of decanol, and 500 m of $H_2SO_4$. The reaction was heated until methanol formation ceased. The reaction was then neutralized with 0.1 N NaOH (100 mL) and extracted. The organic layer was dried over $MgSO_4$, filtered, and dried in vacuo to give 400 g of crude mixture. The fractional distillation was carried out 3 ti to give 98.5 g (45% yield, 100% pure by GC) of a clear colorless oil.
B.P. = 122-124° C/2.0 mm (lit. B.P. = 109° C/1.0 mm)
GC ($R_t$): 14.61
IR (neat, in $cm^{-1}$): 3467.3 (br.s), 1737.5 (s), 1465.5 (s), 1264.9 (s), 1213.02 (s), 1131.2 (s), 1044 (m)
$^1H$ NMR (200 MHz FT, $CDCl_3$ with TMS): δ 4.4 (q,J = 6.9 Hz, 1HO, 4.15 (dt,J = 0.9 Hz,J = 6.5 Hz, 2H), 3.1 (br.s, 1H), (br.s 6.5 Hz, 2H), 1.45 (d,J = 6.9 Hz, 3H), 1.3 (br.s, 14H 0.9 (br.t, 6.6 Hz, 3H).
$^{13}C$ NMR (50 MHz, $CDCl_3$, in ppm): 175.6, 66.7, 65.5, 31.7, 29.4, 29.3, 29.1, 29.0,28.4, 25.7, 22.6, 20.3, 13.9

Preparation of Decyl Lactate by Direct Esterification

A 500 mL one neck round bottom flask equipped with a Dean Stark trap,condensor and nitrogen inlet/outlet was charged with 22.52 g (0.75 moles) lactic acid, 47.46 g (0.98 moles decyl alcohol, 3 ml sulphuric acid and 220 mL toluene. Th mixture was heated to 145° C for 20 hours and water was collected as the reaction proceeded. The acid was neutralized by washing three times with 50 ml saturated sodium bicarbonate solution. The organic layer was collec and dried over magnesium sulfate. Decyl lactate was distilled under high vacuum to yield 24.03 g (12.4% yield) clear colorless liquid. According to GC the product is 97.78% pure.

Preparation of Decyl 2-Sulfooxypropionate Sodium Salt from Decyl Lactate

A 50 mL Schlenk flask equipped with a spin bar was charged with 2.45 g (21 mmole) of $ClSO_3H$. The reaction flask was cooled to ca. 0° C in an ice/water bath under a nitrogen atmosphere and decyl lactate (4.6 g, 20 mmole) was slowly added. After addition was complete, the reaction was maintained at 0° C and was stirred for 15 minutes. A solut of sodium hydroxide in deionized water/ice was added, and was adjusted to 7.0. This water solution was extracted wi ether to remove any organic residue. The water layer was collected and lyophilized to give 8.7 g of a crude white product. The white solid was washed with warm ethanol, filtered and dried to give 6.7 g (100% yield) of white sol
IR (nujol, in $cm^{-1}$): 1740 (s), 1255 (br.s), 1220 (br.s), 11 (s), 1060 (s), 1030 (s), 990 (m), 950 (s)
$^1H$ NMR (100 MHz FT, $D_20$) δ 4.65 (apparent q,J = 6.9 Hz, 1H) 4.0 (m, 2H), 1.55 (br.s, 2H),1.35 (d,J = 6.9 Hz, 3H), 1.15 (br.s, 16H), 0.9 (br.s, 3H)
$^{13}C$ NMR (50 MHz, $D_20$ with TSP in ppm): 175.7, 75.34, 68.73, 34.8, 32.6, 32.5, 32.3, 31.1, 28.67, 25.45, 20.9, 16.6
High Resolution FAB M.S. (from glycerol/$H_20$, (%) relative intensity): m/z 355 (M + Na, 100%)

Example 4

Preparation of Dodecyl 2-Sulfooxypronionate Sodium Salt

Preparation of Dodecyl Lactate

A 1 liter one neck round bottom flask equipped with a distillation apparatus and nitrogen inlet/outlet was charg with 200.0 g (2.69 moles)ethyl lactate, 536 g (3.4 moles) dodecyl alcohol and 0.85 g sulphuric acid. The reaction w refluxed for 12 hours and ethanol was collected as formed. The acid was neutralized by washing three times with 100 m saturated sodium bicarbonate solution. The organic layer collected and dried over magnesium sulfate. Excess dodecy alcohol was removed by high vacuum distillation leaving 27 g (62.1% yield) of clear viscous light gold colored oil. According to GC and the product is 100% pure.
GC (Rt): 17.14

IR (neat, in $cm^{-1}$) : 3460 (br.s), 1735 (s) 1470 (s), 1380 (m 1260 (br.s), 1210 (br.s), 1130 (s), 1040 (m)
$^{1}$H NMR (200 MHz FT, $CDCl_3$ with TMS): $\delta$ 4.3 (m, 1H0, 4.2 (dt = 6.5 Hz,J = 1.4 Hz, 2H) 3.1 (d,J = 6.3 Hz, 1H), 1.7 (br.m 2H), 1.4 (d,J = 6.9 Hz, 3H) 1.26 (br.s, 18H), 0.9 (br.t, J 6.7 Hz,3H)
$^{13}$C NMR (50 MHz, $CDCl_3$ with TMS,in ppm): 176.4, 67.28, 66.3 32.47, 30.18, 30.1, 30.05, 29.9, 29.74, 29.6, 29.09, 26.34 23.24,20.97, 14.65

Preparation of Dodecyl 2-Sulfooxypropionate Sodium Salt fr Dodecyl Lactate

A 100 ml round bottom flask equipped with a spin bar was charged with 6.06 g (52 mmole) of $ClSO_3H$. The reaction flask was cooled to ca. 0°C in an ice/water b and 12.9 g (50 mmole) of dodecyl lactate was slowly added. The addition was slow and reaction temperature was maintai at ca. 0°C. After addition was complete, the reaction was stirred for 15 minutes. To a beaker filled with a solutio of sodium hydroxide (2.08 g) in deionized water/ice was ad the crude reaction mixture. The pH was brought to ca. 7.0 This water solution was extracted with ether to remove any organic residual. The water layer was collected and lyophilized to give 20.1 g of a white solid. From M.S. an $^{1}$H NMR, a trace of unreacted dodecyl lactate is present in the reaction mixture.
IR (nujol, in $cm^{-1}$): 1730 (s),1250 (vs), 1220 (br.s), 1200 (s), 1130 (vs), 1100 (br.s), 1070 (s), 1030 (s), 965 (s) $^{1}$ NMR (100 MHz FT, $D_20$) $\delta$ 4.65 (apparent q, 1H), 3.9 (br.s, 2H), 1.45 (br.s, 2H), 1.35 (d,J = 7.2 Hz, 3H), 1.15 (br.s, 20H), 0.9 (br.s, 3H)
$^{13}$C NMR (50 MHz,$D_20$ with TSP in ppm): 175.2, 74.9, 68.27, 34.48, 32.5, 32.05, 30.84, 28.35, 25.09, 25.45, 22.55, 21. 20.5, 16.2
High Resolution FAB M.S. (from glycerol/$H_20$, (%) relative intensity): m/z (M + Na, 100%)

Example 5

Preparation of Decyl 3 - Sulfooxybutyrate Sodium Salt

Preparation of decyl 3-Hydroxybutyrate

A 100 ml one neck round bottom flask equipped with a Dean Start trap,condensor and nitrogen inlet/outlet was charged with 20.8 g (0.20 moles) 3-hydroxybutyric acid, 63.3 g (0. moles) decyl alcohol, and 0.1 g sulphuric acid. The mixtu was heated to 140°C for 8 hours and water was collected as the reaction proceeded. The acid was neutralized by washi three times with 50 ml saturated sodium bicarbonate soluti The organic layer was collected and dried over magnesium sulfate. Excess decyl alcohol was removed by high vacuum distillation to yield 28.3 g (58% yield) of clear viscous colorless oil. According to GC the product is 89.4% pure.
GC ($R_t$): 15.6
IR (neat, in $cm^{-1}$): 3450 (br.s), 1730 (s), 1470 (s), 1170 (
$^{1}$H NMR (200 MHz FT, $CDCl_3$ with TMS) $\delta$ 4.3 (m, 1H), 4.2 (t,J 6.7 Hz, 2H), 3.5 (-OH,d, J= 3.8 Hz), 2.4 (d,J = 5.9 Hz, 2H 1.9-1.5 (br.s, 21H), 0.9 (br.t, J= 6.7 Hz, 3H)
$^{13}$C NMR (50 MHz, $CDCl_3$ with TMS, in ppm): 170.15, 62.14, 61.59, 40.42, 29.3, 26.93, 26.8, 26.71, 26.65, 25.96, 23.3 20.08, 19.92, 11.48

Preparation of Decyl 3-Sulfooxybutyrate Sodium Salt from Decyl 3-Hydroxybutyrate

A 100 mL round bottom flask equipped with a spin bar was charged with 6.8 g (58.2 mmole) of $ClSO_3H$. The reaction flask was cooled to ca 0°C in an ice/water bath and 14.2 g (58.2 mmole) of decyl 3-hydroxybutyrate was slowly added t ca. 0°C. After addition was complete, the reaction was stirred for 30 minutes. To a beaker filled with a solutio of sodium hydroxide (4.6 g) in deionised water/ice was add the crude reaction mixture. The pH was brought to 7.0. T water solution was extracted with ether to remove any orga residual. The water layer was collected and lyophilized t give 24.7 g of a white solid. The crude material was adde to 2 L beaker equipped with spin bar and 1.2 L of absolute ethanol. The mixture was stirred at room temperature for hours. The white solid was filtered and ethanol was concentrated to give sticky white solid which was dissolve in water and lyophilized to give (98% yield) of white powd
IR (nujol, in $cm^{-1}$): 1730 (s), 1300 (s), 1255 (s) 1200 (s) 1185 (s), 1135 (s), 1080 (s), 1030 (s), 930 (s)
$^{1}$H NMR (100 MHz FT, $D_20$ with TSP in ppm) $\delta$ 4.6 (apparent q overlapped with DHO peak, 1H), 3.9 (t, J = 6.5 Hz, 2H), 2. (dd, J = 6.4 Hz, J = 15.3 Hz, 1H), 2.4 (dd, J = 15.3 Hz, J 6.4 Hz, 3H), 1.5 (br.t, 2H), 1.2 (d, J = 6.4 Hz, 3H), 1.15 (br.s, 16H), 0.9 (br.t, J = 6.4 Hz, 3H)

$^{13}$CNMR (50 M Hz, $D_2O$ with TSP in ppm: 174.62, 75.52, 67.78, 43.94, 34.43, 32.15, 31.89, 30.84, 28.4, 25.11, 22.66, 16.
High Resolution FAB M.S. (from glycerol/$H_20$, (%) relative intensity): m/z 369 (M + Na, 100%)

Example 6

Preparation of Dodecyl 3-Sulfooxybutyrate Sodium Salt

Preparation of Dodecyl 3-Hydroxybutyrate

A 500 mL one neck round bottom flask equipped with a Dean Stark trap, condenser, and a nitrogen inlet/outlet was charged with 72.0 g (69 mmole) of 3-hydroxybutyric acid, 268.5 g (1.45 mole) of dodecyl alcohol, and 0.36 g of sulphuric acid. The mixture was heated to 120°C for 48 ho and water was collected as the reaction proceeded. The ac was neutralized by washing three times with 100 mL saturat $NaHCO_3$ solution. The organic layer was collected and drie over $MgSO_4$ and concentrated. Excess dodecanol was removed high vacuum distillation to yield 117.65% (60% yield) of clear oil (91% pure by GC).
GC (Rt): 18.17
IR (neat, in $cm^{-1}$): 3450 (br.s), 1730 (s), 1465 (s), 1375 (m), 1295 (s), 1180 (s), 1080 (m)
$^1$H NMR (200 MHz, $CDCl_3$ with TMS) δ 4.15 (m, 1H), 4.1 (t, J 6.7 Hz, 2H), 3.15 (br.s, -OH, 1H), 2.4 (apparent t, J = 4 2H), 1.6 (br.m, 2H), 1.25 (br.s, 20H), 0.9 (bt. t, J = 6.8 Hz, 3H)

Preparation of Dodecyl 3-Sulfooxybutyrate Sodium Salt from Dodecyl 3-Hydroxybutyrate

A 50 mL Schlenk flask equipped with a spin bar and additio funnel was charged with 6.4 g (55 mmole) of $ClSO_3H$. The reaction flask was cooled to ca. 0°C in an ice/water bath 13.6 g (50 mmole) of dodecyl hydroxybutyrate was slowly added. The addition was slow and reaction was maintained ca. 0°C. After addition was complete, the reaction was stirred for 15 minutes. To a beaker filled with a solutio of sodium hydroxide (2.2 g) in deionized water/ice was add the crude reaction mixture. The pH was brought to 7.0. T water solution was extracted with ether to remove any orga residual. The water layer was collected and lyophilized t give 20.6 g of a white solid. The white solid was dissolv in warm ethanol, filtered and ethanol was concentrated to give sticky white solid which was dissolved in water and lyphilized to give 18.3 g (98% yield) of white powder.
IR (nujol, in $cm^{-1}$): 1735 (s), 1300 (m), 1255 (vs), 1200 (s 1200 (vs), 1180 (vs), 1080 (s), 1030 (s)
$1^H$ NMR (300 MHz FT,$D_2O$ with TSP) δ 4.8 (apparent q, J = 6.6 Hz, 1H), 4.2 (m,diastereotopec 2H), 1.68 (br.t, J = 6.6 Hz 2H), 1.5 (d, J = 7.2 Hz, 3H), 1.3 (br.s, 20H), 0.9 (br.t, 6.9 Hz, 3H)
$^{13}$C NMR (50 MHz, $D_2O$ with TSP in ppm) 174.6, 75.5, 67.85, 44.1, 34.7, 32.66, 32.59, 32.23, 27.33, 25.35, 22.94, 21.4 16.5
High Resolution FAB M.S. (from glycerol/$H_2O$, (%) relative intensity): m/z 397 (M + Na, 100%)

Example 7

Preparation of 2-Sulfooxyoctanoate

Preparation of Decyl 2-Hydroxyoctanoate

A 250 mL one neck round bottom flask equipped with a Dean Stark trap, condensor and nitrogen inlet/outlet was charge with 20.0 g (0.12 moles) 2-hydroxyoctanoic acid, 79.0 g (0 moles) decyl alcohol, and 624 mg sulphuric acid. The mixt was heated to 140°C for 8 hours and water was collected as the reaction proceeded. The acid was neutralized by washi three times with 50 ml saturated sodium bicarbonate soluti The organic layer was collected and dried over magnesium sulfate. Excess decyl alcohol was removed by high vacuum distillation to yield 28.99 g (77.3% yield) of clear pale yellow liquid. According to GC the product is 95.0% pure.
GC (Rt): 20.0
IR (neat, in $cm^{-1}$): 3500 (s), 1735 (s), 1470 (s), 1210 (s) 1120 (s), 1090 (s)
1H NMR (200 MHz FT, $CDCl_3$ with TMS): δ 4.2 (m, 3H), 3.4 (-br.s, 1H), 1.7 (m, 4H), 1.27 (br.s, 22H), 0.9 (2t, J = 6Hz 6H)
$^{13}$C NMR (50 MHz, $CDCl_3$ with TMS, in ppm): 175.4, 70.21, 65. 34.1, 31.67, 31.3, 29.31, 29.09, 28.83, 28.7, 28.5, 28.36, 25.64, 24.5, 22.44, 22.35, 13.78, 13.73

Preparation of 2-Sulfooxyoctanoate from Decyl 2-Hydroxyoctanoate

A 50 mL Schlenk flask equipped with a spin bar and additio funnel was charged with 1.4 g (12 mmole) of $ClSO_3H$. The reaction flask was cooled to ca. 0 ° C in an ice/water bath 3.0 g (10 mmole) of decyl hydroxyoctanoate was slowly adde The addition was slow and reaction was maintained at ca. 0 After addition was complete, the reaction was stirred for minutes. To a beaker filled with a solution of sodium hydroxide in deionized water/ice was added the crude react mixture. The pH was brought to 7.0. The crude mixture wa lyophilized to give 4.8 g of a white solid. The solid was not readily soluble in water or alcohols (methanol and ethanol). The white solid was washed with cold water and white sticky solid was filtered to give 2.45 g (62% yield) product after drying.
IR (nujol, in $cm^{-1}$): 1745 (s), 1280 (s), 1215 (s), 1140 (s) 970 (s), 930 (s)
Low Resolution M.S.: m/z 425 (M + Na+)

Example 8

Critical Micelle Concentration of Sulfoxy Ester as Measure Surfactancy

Due to energetic reasons, surfactants strive to reduce the contact of their hydrophobic chains in water. At the water/air interface, surfactants adsorb with their hydrophobic chains on the water surface. The adsorption o surfactants results in the lowering of surface tension (Gibb's law). Plotting the interfacial tension against th logarithm of the concentration of the surfactants, one can determine critical micelle concentration (CMC).

The micelle formation property which surfactant active solutes have of forming colloidal-sized clusters in soluti is an important phenomena because it is an interfacial phenomena important for defining detergency and solubilization. Specifically, the critical micelle concentration (CMC) is the concentration at which surfacta creates micelles.

The value of the critical micelle concentration was determined by surface tension using the Wilhemy plate meth The comparison was a comparison between one of the alkyl sulfooxy esters of the invention-(DSB) and a common commerc anionic surfactant (SDS). The results of the CMC calculations are represented in Table 3 below:

Table 3

| Critical Micelle Concentration of Some Surfactants in Aqueous Media | | | |
|---|---|---|---|
| Compound | Solvent | Temperature (° C) | CMC(m |
| Sodium Laurylsulfate (SDS) | $H_20$ | 40 | 8.6 |
| Sodium Decyl 3-Sulfooxybutyrate (DSB) | $H_20$ | 45 | 0.4 |

* as reported at 40 ° C by Flockhart, J. Colloid Sci., 16:48 (1961). The minimum surface tension for DSB was about 30 dyne/cm.

As can be observed from Table 3, the critical micelle concentration (i.e., concentration at which surfactant creates micelles) is much lower for DSB than SDS indicatin that DSB is much more surface active. While not wishing t be bound by theory, it is believed that lower CMC values suggest that lower amounts of DSB need be used to obtain t same surfactancy benefits.

Further, while again not wishing to be bound by theory, th art seems to indicate that as the CMC gets lower, the surfactant is more mild.

Example 9 - Comparison of Krafft Point

The temperature at and above which surfactants begin to fo micelles instead of precipitates is referred to as Krafft point (Tk) and at this temperature the solubility of an io surfactant becomes equal to its CMC. The micelle formatio will increase the solubility.

The Krafft point for DSB was measured to be less than 0 ° C. The surfactant showed a lower Krafft point compared to corresponding surfactant which posses similar hydrophilic group (i.e., SDS). While not wishing to be bound by theor it is believed that the effect of substitution of ester gr on the hydrocarbon chain may result in an increase in the solubility.

Lower Krafft point may result in additional benefits depending on the desired specifications of the formulation which the surfactant is being used. For example, surfacta with lower Krafft points are more stable in aqueous system In addition, it is easier to formulate such surfactants in multi-electrolyte systems since they will be more tolerant salt.

Example 10

Foam Ability of Alkyl Sulfooxyalkanoates

Foam is an important attribute in many consumer products (e.g. cosmetic products). Foam is one of the dominant factors that determines the commercial value of products s as shampoo, soap, etc. Also, acceptability of many consum products is closely related to the quality and texture of foam they produce (psychological aspect).

Since most of the foaming data on surfactants is typically obtained by the Ross-Miles method (Ross, J. and Miles G.D. Am. Soc. for Testing Material Method (D 1173-53), Philadelphia, Pa. (1953); Oil and Soap, 62:1260 (1958)), t foaming ability of these surfactants was also acquired usi this method.

In the Ross-Miles method, 200 mL of a solution of surfacta contained in a pipette of specified dimensions with a 2.9-mm-i.d. orifice is allowed to fall 90 cm onto 50 mL of the same solution contained in a cylindrical vessel maintained at a given temperature (often 60 ° C) by means of water jacket. The height of the foam produced in the cylindrical vessel is rad immediately after all the soluti has run out of the pipette (initial foam height) and then again after a given amount of time (generally, 5 min).

Using this method, the foam production (measured initially and foam stability (the height after 10 minutes) are reported. All of the foaming was achieved at 45 ° C in wate with 0 ppm hardness. The foam height is represented in mm illustrated in Table 4.

Table 4

| Ross-Miles Foam Height Test | | | | |
|---|---|---|---|---|
| Compound | Initial Height | | Final Height (after 10 minute | |
| | 0.05% | 0.1% | 0.05% | 0.1% |
| DSB | negligible | 109 | 0 | 36 |
| LSB | 141 | ---* | 84 | ---* |
| DSP | negligible | 143 | negligible | negligib |
| LSP | 139 | 177 | 124 | 153 |

* not measured

As shown in Table 4, DSB produced negligible foam at a low concentration (0.05% active) but gave a respectable but apparently unstable foam at 0.1% active solution. However by adding two more methylene groups onto the hydrophobe ch (LSB), we obtained a respectable foam even at a lower concentration. It appears that the foam increases with an increase in the number of carbon chain.

As can be observed from Table 4, at least some of the alkylsulfooxy alkanoates of the invention (i.e., LSB and L show good foaming at concentrations as low as 0.05 while a showed good foaming at concentrations of 0.1%.

Example 11

Hardness Sensitivity Test

The calcium ion stability of alkyl sulfooxyalkanoates was measured by a modified Hart method (Witkes, et al. J. Ind. Eng. Chem., 29, 1234-1239 (1937). The surfactant solution was titrated with a calcium ion solution. The endpoint wa determined by visual observation of the cloudiness of the surfactant solution.

Many surfactants like fatty soap are known to chelate to calcium ion to form calcium salts which are usually insolu in aqueous media. This will lead to the loss of their surfactant properties. Calcium "insensitive" surfactants have unique advantageous properties for many applications such as a formulation for a liquid cleanser. In the case the sulfate analogs, we noticed that a large amount of calcium ion was

added up before precipitation was seen. F LSP and LSB, we did not reach the precipitation limit even levels well over an order of magnitude higher than the precipitation limit for sodium dodecyl sulfate (SDS). The calcium ion stability results are represented in Table 5.

Table 5

| Calcium Ion Sensitivity Test | | |
|---|---|---|
| Compound | Head Group | $Ca^{+2}$ Required for ppt |
| Sodium dodecylsulfate (SDS) | sulfate | 120 ppm |
| Sodium decyl 3-sulfooxybutyrate (DSB) | sulfate | >1550 ppm |
| Sodium lauryl 3-sulfooxybutyrate (LSB) | sulfate | >2500 ppm |
| Sodium decyl 2-sulfooxypropionate (SDSP) | sulfate | ca. 2126 ppm |
| Sodium dodecyl 2-sulfooxypropionate (SLSP) | sulfate | >2500 ppm |
| Sodium decyl 3-sulfopropionate (SDSfP) | sulfonate | 650 ppm* |
| Sodium dodecyl 3-sulfopropionate (SLSfP) | sulfonate | 160 ppm* |
| Sodium lauryl isethionate (SLI) | sulfonate | 51 ppm |

*Value measured by Hikato et al., Bull of Chem. Soc., Java 43:158 (1970)

A comparison of the calcium ion sensitivity of LSB and DSB showed that the DSB was slightly less sensitive than LSB. These compounds differ by the number of carbon atoms in th alkyl chain. A similar trend is seen with propionate analogs. Interestingly, Hikota, et al., observed a revers relationship in his alkyl sulfoalkanoates (sulfonates not sulfates). The calcium ion stability of the surfactant decreased with an increase in the number of carbon atoms. similar relationship was also seen in SLI analogs (Hikota al., Bull of Chem. Soc. Japan, 43: 158-161 (1970))

In general, it can be seen that the alkyl sulfooxyalkanoat used in the compositions of the invention are much less calcium sensitive than corresponding sulfate analogs or th the commercially available SDS. This offers a clear advantage in terms of the ability to formulate and use the novel surfactants of the invention in hard (i.e., calcium containing) water.

Example 12

In vitro "Mildness" Test

Assessing Mildness

Many factors have been reported to have an influence on sk irritation such as removal of skin lipids, loss of natural occuring hygroscopic materials in the stratum corneum, adsorption, protein denaturation, and epidermial lyposomal injury. Although there are many hypotheses regarding skin irritation, it is generally believed that surfactants beco irritants because they penetrate the stratum corneum which a "barrier" and then react with the inner cells of the epidermis.

Traditionally, the study of percutaneous absorption has focused on measuring the diffusion of chemicals (e.g. surfactants through stratum corneum). Diffusion through a organ as complex as skin and its associated adnexal appendages is challenging to measure, model, and predict. Another challenge of cutaneous metabolism is to assess the irritating potential, toxicity, and therapeutic potential the penetrating compounds.

In vivo, the skin metabolism and percutaneous absorption a very difficult to measure. Finding adequate detection methods and setting up proper experiments are not easy tas In vitro studies however are used because of the simplicit of the experimental conditions. We can also minimize or eliminate the use of animals.

We have obtained information on mildness potentials of the surfactant by carrying out in vitro tests which have been demonstrated to correlate well with in vivo tests.

In vitro protein interaction test

Van Scott and Lyon (Van Scott et al., J. Invest. Dermatol. 21:199-203 (1953)) concluded from their work that protein denaturation was an important mechanism in the production dermatitis. Blohn (Blohm, S.G.,

Acta. Derm. Venerol., 37:269-275 (1957)) showed the effect of denaturating a purified protein (ovalbumin) system using sodium dodecyl sulfate (SDS). This type of demonstration showed a relationship between skin irritation and protein denaturin effects of surfactants.

An in vitro "Zein dissolution test" may be used to measure mildness. Specifically, the lower the amount of zein prot dissolved, the milder the surfactant is. Conversely, the more zein dissolved, the more irritating the surfactant is SDS, which is known to be an irritating surfactant, dissol 85% of the zein as seen in table 6. by contrast, the amou of zein dissolved by the alkyl sulfooxy alkanoates used in the compositions of the invention was equivalent to that dissolved by SLI, a known mild active ingredient.

Table 6

| Zein Dissolution Test | |
|---|---|
| Compound | % Zein Dissolved in H |
| Blank (control) | 9% |
| sodium dodecylsulfate (SDS) | 86% |
| Sodium laurylisethionate (SLI) (sulfonate) | 55% |
| Sodium lauryl sulfooxybutyrate (LSB) | 54% |
| Sodium decyl sulfooxybutyrate (DSB) | 54% |

As shown by the comparison of LSB and DSB, the dissolution Zein appears to be independent of the hydrophobe chain length.

## Example 13

Sulfoxy Mono- or Di-Ester is Used in a Toilet Soap Bar

| Ingredients | % by Weight |
|---|---|
| C8-24 fatty acid soap | 30-95% |
| Alkyl sulfooxy alkanoate | 0-45% |
| Alkyl sulfate | 0-5% |
| Moisturizer (e.g. Sorbitol or Glycerin) | 0.1-10% |
| Water soluble polymer (e.g. Cellulase or Polyacrylates) | 0-10% |
| Sequestering agents (e.g. citrate) | 0.1-0.5% |
| Dye stuff | < 0.1% |
| Optical brighteners | < 0.1% |
| Whitening agents | 0.1-0.4% |
| Fragrance | 0.1-2.0% |
| Water | Balance |

Example 14

Sulfate Ester is Used in a Facial/Body Cleanser Compositio

| Ingredients | % by Weight |
|---|---|
| C8-24 fatty acid salt (e.g. triethanolamine) | 1-45% |
| Alkyl sulfooxy alkanoate | 10-75% |
| Alkyl sulfate | 0-20% |
| Coactive surfactant (e.g. cocoamidobetaine) | 1-15% |
| Moisturizer (e.g. sorbitol) | 0.1-15% |
| Refattying alcohol | 0.5-5% |
| Water soluble polymer | 0-10% |
| Thickener | 0-15% |
| Conditioner (e.g. quaternized cellulose) | 0-0.5% |
| Sequestering agents (e.g. citrate) | 0.1-0.4% |
| Dye stuff | < 0.1% |
| Optical brighteners | < 0.1% |
| Whitening agents | 0.1-0.4% |
| Fragrance | 0.1-3.0% |
| Preservatives | 0-0.2% |
| Water | Balance |

Example 15

Sulfate Ester is Used in a Toothpaste Composition

| Ingredients | % by Weight |
|---|---|
| Synthetic surfactants (sodium lauryl sulfate) | 1.5% |
| Alkyl sulfooxy alkanoate | 0-10% |
| Alkyl sulfate | 0-1% |
| Abrasive (e.g. silic acid/$CaCO_3$) | 20-55% |
| Active ingredients (e.g., Pyrophosphates) | 0.1-2% |
| Humectant (glycerin, sorbitol) | 10-45% |
| Thickeners (cellulose derivatives) | 0-3% |
| Sequestering agents (e.g. citrate) | 0.1-04% |
| Flavoring agents | 0.5-2% |
| Sweeteners | < 0.5% |
| Dye stuff | < 0.1% |
| Water | Balance |

## Claims

**1.** An alkyl sulfooxy alkanoate molecule having the formula

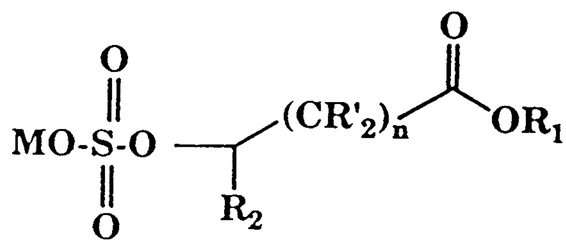

wherein:

M is selected from the group consisting of hydrogen, an alkali-metal, an alkaline earth metal, a cationic amino acid and salt forming cations;

R' is hydrogen, a straight chain alkyl group having 1-30 carbons, a branch-chained alkyl group having 4 to 30 carbons, an organic moiety containing a functional group or a combination of any of these groups.

$R_2$ is hydrogen, a straight chain alkyl group having 1 to 30 carbons wherein any of the carbons may be substituted with a functional group; a saccharide group; a branched chain alkyl having 4 to 30 carbons; a straight or branched chain alkyl aryl group wherein the alkyl group may comprise 1-18 carbon atom and wherein said alkyl aryl group may be condensed with a 2-5 carbon alkylene oxide; an aliphatic group having 6 to 30 carbons condensed with a 2-5 carbon ethylene oxide; a straight or branch-chained fluoroalkyl group having 5 to 23 carbons; or a carbocyclic containing group; wherein any of the alkyl groups described above may be linked by an ester group, amide, quaternary ammonium or a heteroatom such as sulphur, oxygen or nitrogen; and

$R_1$ is the same or different than $R_2$.

n = 0 to 50; and

which is capable of forming a hydroxy carboxylic acid ester, a hydroxy carboxylic acid, a sulfooxy carboxylate and/or alcohol when the surfactant is metabolized or hydrolyzed.

**2.** A molecule according to claim 1, wherein $R_1$ is $C_{10}$ $H_2$ $R_2$ is $CH_3$ and n = 0 (DSP).

**3.** A molecule according to claim 1, wherein $R_1$ is $C_{12}$ $H_2$ $R_2$ is $CH_3$ and n = 0 (LSP).

**4.** A molecule according to claim 1, wherein $R_1$ is $C_{10}H_{21}$ $R_2$ = $CH_3$, R' = hydrogen and n = 1 (DSB).

**5.** A molecule according to claim 1, wherein $R_1$ is $C_{12}H_{25}$ $R_2$ = $CH_3$, R' = hydrogen and n = 1 (LSB).

**6.** A molecule according to claim 1, wherein $R_1$ = $C_8H_{17}$, = $CH_3$, and n = 0 (OSP).

**7.** A molecule according to claim 1, wherein $R_1$ = $C_{10}H_{21}$, = $C_6H_{13}$, and n = 0 (DSO).

**8.** A molecule according to any of the preceding claims, wherein the the straight chain alkyl group of $R_2$ is substituted with a functional group selected from the group consisting of esters, hydroxy groups, halides, and substituted or unsubstituted hydroxy fatty amides

**9.** A molecule according to any of the preceding claims, wherein the saccharide of $R_2$ is a monosaccharide or oliyosaccharide unsubstituted or substituted with an alkyl chain having 1 to 30 carbons.

**10.** A composition for topical application to the human skin, wherein the composition comprises a molecule according to any of the preceding claims in a physiologically acceptable carrier.

**11.** A composition according to claim 10, wherein the composition is a soap bar composition.

**12.** A composition according to claim 10, wherein the composition is a facial or body cleansing composition

**13.** A composition according to claim 10, wherein the composition is a shampoo composition.

**14.** A composition according to claim 10, wherein the composition is a conditioner composition.

**15.** A composition according to claim 10, wherein the composition is a cosmetic composition.

**16.** A composition according to claim 10, wherein the composition is a dental composition.

**17.** A composition according to claim 10, wherein the composition is an underarm deodorant/anti-perspirant composition.

**18.** A method for producing a molecule according to any of claims 1-9, comprising:
(a) preparing a desired hydroxy carboxylic acid or hydroxy carboxylic acid ester;
(b) sulfating the hydroxy carboxylic acid and/or the ester;
(c) neutralizing the sulphated hydroxy carboxylic ac and/or ester; and
(d) recovering.

**19.** A method according to claim 18 wherein the hydroxy carboxylic acid ester is a lactone.

**20.** A method according to claim 18 or claim 19, wherein free alcohol is a by-product of the reaction for preparing the hydroxy carboxylic acid and/or the aci ester.

**21.** A method according to any of claims 18-20, wherein t sulphation is done using vacuum distillation and results in substantially pure yields of hydroxy carboxylic acid and/or the carboxylic acid ester.

**22.** A method according to any of claims 18-21, wherein t hydroxy carboxylic acid and/or the ester of step (a) prepared by direct or transesterification.

**23.** A method according to any of claims 18-22, wherein t sulphation of Step (b) is conducted at a temperature from about -80° to about 120°C.

**24.** A method according to claim 23, wherein the sulphati is conducted at a temperature of about -20 to about 35°C.

**25.** A method according to claim 23, wherein the sulphati is conducted at a temperature of about 0 to about 5°

**26.** A method according to any of claims 18-25, wherein t sulfating of step (b) is accomplished using chlorosulfonic acid.

**27.** A method according to any of claims 18-26, wherein t sulphation is accomplished under anhydrous condition

**28.** A method according to claim 27, wherein sulphation i accomplished under a nitrogen blanket at atmospheric pressure.

**29.** A method according to any of claims 18-28, additiona comprising the use of an acid scavenger.

**30.** A method according to any of claims 18-29, wherein t sulphation step runs from about 5 minutes to about 2 hours.

**31.** A method according to any of claims 18-30 in which a solvent is additionally used during the sulphation step.

**32.** Use of a molecule according to any of claims 1-9 in composition for topical application to the human ski

European Patent Office

# PARTIAL EUROPEAN SEARCH REPORT

which under Rule 45 of the European Patent Convention shall be considered, for the purposes of subsequent proceedings, as the European search report

Application Number

EP 92 20 2054

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 225 673 (J.H. WERNTZ et al.)<br>* Page 3; column 2, claims 1-10; pages 1-2; examples I-III; page 3, column 1, lines 49-53; page 3, column 2, paragraph 2 *<br>--- | 1 | C 07 C 305/06<br>A 61 K 7/48 |
| X | FETTE, SEIFEN, ANSTRICHMITTEL, vol. 67, no. 9, 1965, pages 677-682, Hamburg, DE; H. BERTSCH et al.: "Über die Sulfatierung von 12-Hydroxyoctadecen-(9)-säure-(1)-estern und 12-Hydroxyoctadecansäure-(1)-estern"<br>* Page 682, column 1, paragraph 3; page 682, column 2, paragraphs 1-4 *<br>--- -/- | 1 | |

TECHNICAL FIELDS SEARCHED (Int. Cl.5)

C 07 C

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet -C-

| Place of search | Date of completion of the search | Examiner |
|---|---|---|
| THE HAGUE | 08-10-1992 | FINK D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application Number

EP 92 20 2054

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| X | CHEMICAL ABSTRACTS, vol. 83, no. 14, 6th October 1975, page 207, column 1, abstract no. 117596d, Columbus, Ohio, US; G. CZICHOCKI et al.: "Anion-active surfactants from 10-undecenoic acid", & TENSIDE DETERG. 1974, 11(6), 298-305 * Abstract * --- | 1 | |
| X | CHEMICAL ABSTRACTS, vol. 73, no. 17, 26th October 1970, page 314, column 1, abstract no. 87381d, Columbus, Ohio, US; H. KIIK: "Synthesis of glycolic acid esters and their sodium sulfates", & EESTI NSV TEAD. AKAD. TIOM., KEEM., GEOL. 1970, 19(1), 42-5 * Abstract and RN (29238-01-5), (28801-82-3), (28801-81-2), (28330-51-0) * --- | 1 | TECHNICAL FIELDS SEARCHED (Int. Cl.5) |
| X | CHEMICAL ABSTRACTS, vol. 95, no. 16, 19th October 1981, page 324, column 1, abstract no. 137823b, Columbus, Ohio, US; & SU-A-834 356 (KOMMUNAR MINING-METALLURGICAL INSTITUTE) 30-05-1981 * Abstract * --- | 1 | |
| X | CHEMICAL ABSTRACTS, vol. 116, no. 20, 18th May 1992, page 126, column 1, abstract no. 196611f, Columbus, Ohio, US; A.A. EL-SAWY et al.: "Surfactants made of 2-hydroxy fatty acids. Part II. 2-Hydroxy fatty acids and the hydroxy alkanesulfonate esters" & MAGY. TEXTILTECH. 1991, 44(5), 201-3 * Abstract and RN (140858-49-7), (140842-40-6), (140842-39-3), (140842-38-2), (140842-37-1), (140842-36-0) * --- | 1 | |
| P,X | WO-A-9 111 504 (HENKEL KOMMANDITGESELLSCHAFT AUF AKTIEN) * Page 14; claim 1; pages 12-13; examples 1,2; page 3, last paragraph - page 4, paragraph 1 * --- -/- | 1-6 | |

EPO FORM 1503 03.82 (P0410)

European Patent Office

# PARTIAL EUROPEAN SEARCH REPORT

Application Number
EP 92 20 2054

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | BIOCHEMISTRY, vol. 28, no. 4, 21st February 1989, pages 1604-1612, Columbus, Ohio, US; J.A. PELISKA et al.: "Sulfuryl transfer catalyzed by pyruvate kinase" * Page 1605, column 1, paragraph 7 - column 2, paragraph 1; page 1607, paragraph 5 * --- | 1 | |
| X | US-A-3 595 903 (C.C. GRECO) * Column 3 - column 4, examples 1,4,5 * --- | 1 | |
| X | CHEMICAL ABSTRACTS, vol. 113, no. 2, 9th July 1990, page 50, column 2 - page 51, column 1, abstract no. 7520e, Columbus, Ohio, US; & EP-A-342 815 (IMPERIAL CHEMICAL INDUSTRIES PLC) 23-11-1989 * Abstract and RN (127414-24-8) * --- | 1 | TECHNICAL FIELDS SEARCHED (Int. Cl.5) |
| X | CHEMICAL ABSTRACTS, vol. 107, no. 15, 12th October 1987, page 210, column 2, abstract no. 128595t, Columbus, Ohio, US; S.K. BAINS et al.: "Metabolism in the rat of potassium nonane-5-sulfate, a symmetrical anionic surfactant", & XENOBIOTICA 1987, 17(6), 709-23 * Abstract and RN (110326-62-0) * --- | 1 | |
| X | CHEMICAL ABSTRACTS, vol. 104, no. 17, 28th April 1986, page 659, column 1, abstract no. 148526n, Columbus, Ohio, US; & DE-A-3 404 336 (BASF AG) 08-08-1985 * Abstract and RN (101416-37-9) * --- -/- | 1 | |

European Patent Office

# PARTIAL EUROPEAN SEARCH REPORT

Application Number
EP 92 20 2054

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 102, no. 1, 7th January 1985, page 229, column 2, abstract no. 2394e, Columbus, Ohio, US; A.M.V. CRESCENZI et al.: "Purification and some properties of the D-lactate-2-sulfatase of Pseudomonas syringae GG", & BIOCHEM. J. 1984, 223(2), 487-94<br>* Abstract and RN (93713-87-2), (93633-76-2), (93633-75-1), (3233-46-3) *<br>--- | 1 | |
| X | CHEMICAL ABSTRACTS, vol. 100, no. 25, 18th June 1984, page 163, column 2, abstract no. 204670m, Columbus, Ohio, US; J.L. MAGGS et al.: "Octan-2-sulfate degradation in the isolated perfused rat liver", & BIOCHEM. PHARMACOL. 1984, 33(5), 827-9<br>* Abstract *<br>--- | 1 | TECHNICAL FIELDS SEARCHED (Int. Cl.5) |
| X | CHEMICAL ABSTRACTS, vol. 99, no. 10, 5th September 1983, page 98, column 1, abstract no. 72609x, Columbus, Ohio, US; & CS-A-205 794 (J. BEZEMEK et al.) 30-12-1982<br>* Abstract and RN (42808-36-6) *<br>--- | 1 | |
| X | CHEMICAL ABSTRACTS, vol. 96, no. 6, 8th February 1982, page 73, column 2, abstract no. 36661n, Columbus, Ohio, US; & EP-A-38 620 (IMPERIAL CHEMICAL INDUSTRIES LTD) 28-10-1981<br>* Abstract and RN (80501-50-4) *<br>--- | 1 | |
| X | CHEMICAL ABSTRACTS, vol. 88, no. 3, 16th January 1978, page 534, column 2, abstract no. 21654y, Columbus, Ohio, US; F.M. FOWKES et al.: "Catalysis by cations in cores on non-aqueous micelles", & MICELLIZATION, SOLUBILIZATION, MICROEMULSIONS, (PROC. INT. SYMP.) 1977, 2, 665-73<br>* Abstract and RN (65210-02-8), (65180-93-0) *<br>--- -/- | 1 | |

EPO FORM 1503 03.82 (P0410)

European Patent Office

# PARTIAL EUROPEAN SEARCH REPORT

Application Number
EP 92 20 2054

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| X | CHEMICAL ABSTRACTS, vol. 86, no. 21, 23rd May 1977, page 92, column 2, abstract no. 151061x, Columbus, Ohio, US; B. BURKE et al.: "The biodegradation of the surfactant undecyl sulfate", & XENOBIOTICA 1976, 6(11), 667-78<br>* Abstract and RN (19794-52-6) *<br>--- | 1 | |
| X | CHEMICAL ABSTRACTS, vol. 80, no. 15, 15th April 1974, page 123, column 2, abstract no. 79609p, Columbus, Ohio, US; N. TUDBALL et al.: "Inhibition of the L-serine O-sulfate-degrading system of pig liver and the topography of its active site", & EUR. J. BIOCHEM. 1973, 40(1), 25-9<br>* Abstract and RN (52055-97-7), (52055-96-6) *<br>--- | 1 | TECHNICAL FIELDS SEARCHED (Int. Cl.5) |
| X | CHEMICAL ABSTRACTS, vol. 80, no. 4, 28th Jauary 1974, page 86, column 1, abstract no. 16323v, Columbus, Ohio, US; & DE-A-2 300 015 (CIBA-GEIGY AG) 26-07-1973<br>* Abstract *<br>--- | 1 | |
| X | CHEMICAL ABSTRACTS, vol. 74, no. 15, 12th April 1971, page 270, column 2, abstract no. 74447a, Columbus, Ohio, US; J. OTTERY et al.: "Metabolism of dodecyl sulfate in the rat. Nonenzymic liberation of sulfate and gamma-butyrolactone from the major metabolite, butyric acid 4-sulfate", & LIFE SCI. 1970, 9(23), 1335-40<br>* Abstract and RN (16899-85-7) *<br>--- | 1 | |
| X | CHEMICAL ABSTRACTS, vol. 70, no. 13, 31st March 1969, page 188, column 1, abstract no. 55902w, Columbus, Ohio, US; W.H.B. DENNER et al.: "Metabolism of potassium dodecyl sulfate-35S in the rat", & BIOCHEM. J. 1969, 111(1), 43-51<br>* Abstract and RN (23227-11-4) *<br>----- | 1 | |

EP 92 30 3054

-C-

INCOMPLETE SEARCH

Claims searched completely: 2-32

Claim searched incompletely: 1

As the drafting of the claims is not clear and concise (Art. 83-84 EPO) (see for example the definition of $R^1$ =..., "an organic moiety containing a functional group", ..., or the definition of $R_1$ = "the same or different than $R_2$"), a complete search is not possible on economic grounds (see Guidelines for examination in the EPO, part B, chapter III,2).
Thus, the search and the search report have been limited to the following cases:

$R^1$ = H, alkyl (straight chain or branched)

$R_1$ = $R_2$ (generically, but not necessarily with respect to substituted groups)